(19) Europäisches Patentamt
European Patent Office
Office européen des brevets

(11) Publication number: **0 277 661 B1**

(12) **EUROPEAN PATENT SPECIFICATION**

(45) Date of publication of patent specification: **16.09.92**

(51) Int. Cl.5: **C07D 471/14**, C07D 471/20, //(C07D471/14,235:00,221:00, 209:00),(C07D471/20,235:00, 221:00,209:00)

(21) Application number: **88101870.9**

(22) Date of filing: **27.07.84**

(60) Publication number of the earlier application in accordance with Art.76 EPC: **0 133 309**

The file contains technical information submitted after the application was filed and not included in this specification

(54) **Imidazopyrrolopyridines and condensed derivatives thereof useful in the preparation of herbicidal compounds.**

(30) Priority: **02.08.83 US 519603**

(43) Date of publication of application:
**10.08.88 Bulletin 88/32**

(45) Publication of the grant of the patent:
**16.09.92 Bulletin 92/38**

(84) Designated Contracting States:
**AT BE CH DE FR GB IT LI NL SE**

(56) References cited:
**EP-A- 0 041 623**

(73) Proprietor: **AMERICAN CYANAMID COMPANY**
**1937 West Main Street P.O. Box 60**
**Stamford Connecticut 06904-0060(US)**

(72) Inventor: **Los, Marinus**
**107 Drummond Drive**
**Pennington New Jersey 08534(US)**

(74) Representative: **Wächtershäuser, Günter, Dr.**
**Tal 29**
**W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to novel imidazopyrrolopyridine derivatives and imidazopyrroloquinoline derivatives which can be used for preparing novel, herbicidally effective compounds having a structure depicted by formula I below which are subject of EP-A-133 309, the parent application of the present divisional application:

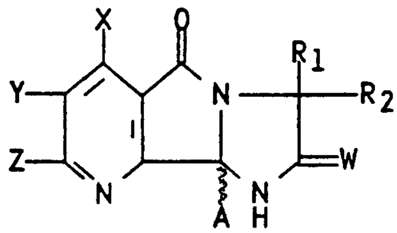

(I)

wherein

$R_1$ and $R_2$ each represent $C_1$-$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$-$C_6$ cycloalkyl ring optionally substituted with methyl;

W is sulfur;

A is hydrogen, hydroxyl, $C_3$-$C_6$ alkenyloxy, $C_3$-$C_6$ alkynyloxy, $C_1$-$C_6$ alkylthio, $NR_{13}R_{14}$ or $C_1$-$C_6$ alkoxy optionally substituted with phenyl, halophenyl, $C_1$-$C_3$ alkylphenyl, $C_1$-$C_3$ alkoxyphenyl or di-$C_1$-$C_3$ alkylaminophenyl;

$R_{13}$ is hydrogen, $C_1$-$C_4$ alkyl optionally substituted with phenyl, halophenyl, $C_1$-$C_3$ alkylphenyl or $C_1$-$C_3$ alkoxyphenyl;

$R_{14}$ is hydrogen or $C_1$-$C_4$ alkyl;

X is hydrogen, halogen or methyl;

Y and Z are each hydrogen, halogen, $C_1$-$C_6$ alkyl, $C_1$-$C_4$ hydroxyalkyl, $C_1$-$C_6$ alkoxy, $C_1$-$C_4$ alkylthio, phenoxy, $C_1$-$C_4$ haloalkyl, $OCF_2CHF_2$,$OCF_3$, $OCHF_2$, nitro, cyano, $NR_4R_5$, $C_3$-$C_8$ straight or branched alkenyloxy optionally substituted with one to three halogens, $C_3$-$C_8$ straight or branched alkynyloxy optionally substituted with one to three halogens, or phenyl optionally substituted with one $C_1$-$C_4$ alkyl, $C_1$-$C_4$ alkoxy or halogen;

$R_4$ is hydrogen or $C_1$-$C_4$ alkyl;

$R_5$ is $C_1$-$C_4$ alkyl;

And, when taken together, Y and Z may form a ring in which YZ is represented by

(1) the structure: $-(CH_2)_n-$, where n is an integer of 2, 3 or 4, provided that X is hydrogen; or

(2) by the structure:

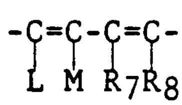

where, L, M, $R_7$ and $R_8$ each represent hydrogen, halogen, $C_1$-$C_4$ alkyl, $C_1$-$C_4$ alkoxy, $C_1$-$C_4$ alkylthio, $C_1$-$C_4$ alkylsulfonyl, $C_1$-$C_4$ haloalkyl, $NO_2$, CN, phenyl, phenoxy, amino, $OCF_3$, $OCHF_2$, $OCF_2CHF_2$, $C_1$-$C_4$ alkylamino, dialkyl($C_1$-$C_4$)amino, chlorophenyl, methylphenyl, $C_3$-$C_8$ straight or branched alkenyloxy optionally substituted with one to three halogens, $C_3$-$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$-$C_4$ alkyl or $C_1$-$C_4$ alkoxy; or

(3) by the structures:

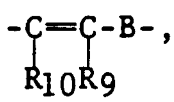, 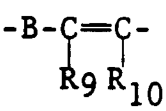, 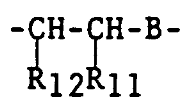, or 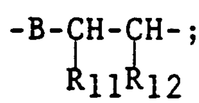;

where B is oxygen or sulfur; $R_9$ and $R_{10}$ each represent hydrogen, halogen, phenyl, or $C_1$-$C_4$ alkyl; $R_{11}$ and $R_{12}$ each represent hydrogen, $C_1$-$C_4$ alkyl or phenyl

and when $R_1$ and $R_2$ are not the same, the cis- or trans-isomers or mixtures thereof or the optical isomers (cis- or trans- or mixtures thereof).

As used in the present specification and claims, the term "halogen" means F, Cl, Br or I, unless otherwise specified.

It should, of course, be understood that when $R_1$ and $R_2$ are not the same, the formula (I) compounds can exist as both the cis- and trans-isomers. These can be illustrated as follows:

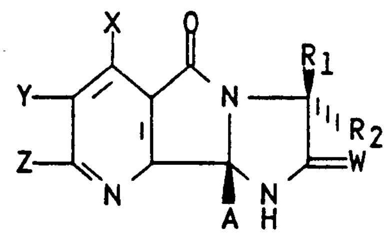

(Ia) cis, and

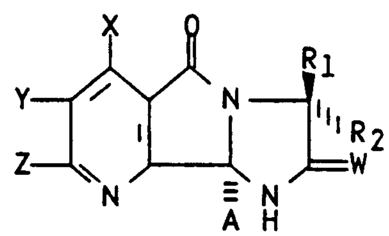

(Ib) trans.

Especially preferred compounds of the EP-A-133 309 are more precisely illustrated by formulas II, III, IV, V, VI, VII and VIII shown below.

Preferred dihydroimidazopyrrolopyridine compounds of this invention are depicted by formula (II), hereinafter illustrated:

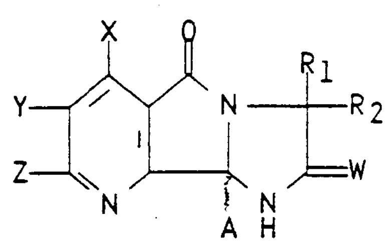

(II)

wherein A, $R_1$, $R_2$, W and X are as defined in reference to formula I above; Y and Z each, independently, represent hydrogen, halogen, $C_1$-$C_6$ alkyl, $C_1$-$C_6$ alkoxy, CN, $NO_2$, $OCF_3$, $OCHF_2$, $OCF_2CHF_2$, phenoxy, $C_1$-$C_4$ haloalkyl, $C_1$-$C_4$ alkylthio, $C_1$-$C_4$ hydroxyalkyl, $NR_4R_5$, $C_3$-$C_8$ straight or branched alkenyloxy optionally substituted with one to three halogens, $C_3$-$C_8$ straight or branched alkynyloxy optionally substituted with one to three halogens, or phenyl optionally substituted with one $C_1$-$C_4$ alkyl, $C_1$-$C_4$ alkoxy or halogen; $R_4$ is hydrogen or $C_1$-$C_4$ alkyl; $R_5$ is $C_1$-$C_4$ alkyl; and when taken together Y and Z may form a ring in which YZ

are represented by the structure: $-(CH_2)n-$, where n is an integer of 2, 3 or 4; and when $R_1$ and $R_2$ are not the same, the cis- or trans-isomers or mixtures thereof or the optical isomers (cis- or trans- or mixtures thereof).

Preferred dihydroimidazopyrroloquinolines are illustrated by formula (III):

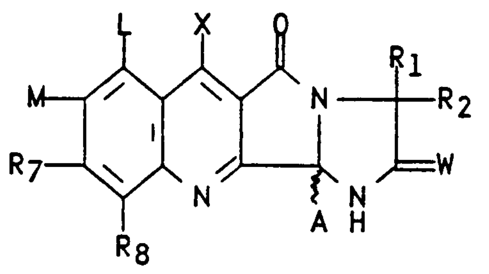

(III)

wherein A, $R_1$, $R_2$, W and X, are as defined above in reference to formula I, and L, M, $R_7$ and $R_8$ represent hydrogen, halogen, $C_1$-$C_4$ alkyl, $C_1$-$C_4$ alkoxy, $C_1$-$C_4$ alkylthio, $C_1$-$C_4$ alkylsulfonyl, $C_1$-$C_4$ haloalkyl, $NO_2$, CN, phenyl, phenoxy, amino, $CF_3$, $OCHF_2$, $OCF_2CHF_2$, $C_1$-$C_4$ alkylamino, dialkyl($C_1$-$C_4$)amino, chlorophenyl, methylphenyl, $C_3$-$C_8$ straight or branched alkenyloxy optionally substituted with one to three halogens, $C_3$-$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$, may represent a substituent other than hydrogen, halogen, $C_1$-$C_4$ alkyl or $C_1$-$C_4$ alkoxy; and when $R_1$ and $R_2$ are not the same, the cis-or trans-isomers or mixtures thereof or the optical isomers (cis- or trans- or mixtures thereof).

Preferred dihydroimidazopyrrolothieno- and furo[3,2-b]pyridines and dihydroimidazopyrrolodihydrothieno- and furo[3,2-b]pyridines are, respectively, illustrated by formulas (V) and (VI), shown below:

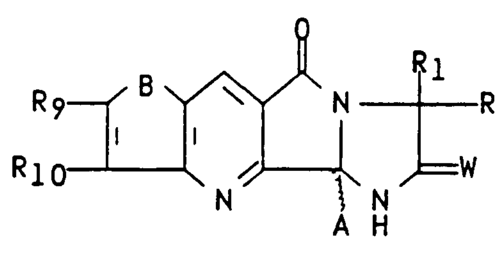

(V) and,

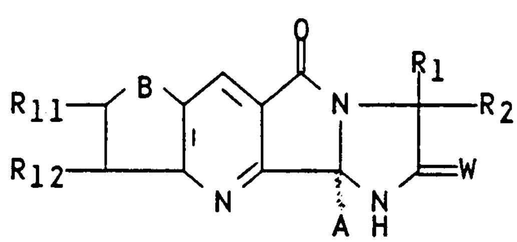

(VI)

wherein A, $R_1$, $R_2$, W and B, are as defined above in reference to formula I; $R_9$ and $R_{10}$ each represent hydrogen, halogen, $C_1$-$C_4$ alkyl or phenyl; and $R_{11}$ and $R_{12}$ each represent hydrogen, $C_1$-$C_4$ alkyl or phenyl; and when $R_1$ and $R_2$ are not the same, the cis- or trans-isomers or mixtures thereof or the optical

isomers (cis- or trans- or mixtures thereof).

The preferred dihydroimidazopyrrolothieno-and furo[2,3-b]pyridines and the dihydroimidazopyrrolodihydrothieno- and furo[2,3-b]pyridines are, respectively, depicted by formulas (VII) and (VIII) illustrated as follows:

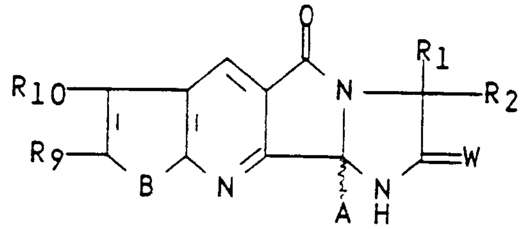

(VII) and,

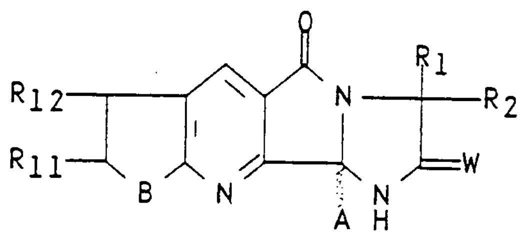

(VIII)

wherein A, $R_1$, $R_2$, W and B, are as defined above in reference to formula I, $R_9$ and $R_{10}$ each represent hydrogen, halogen, $C_1$-$C_4$ alkyl or phenyl; $R_{11}$ and $R_{12}$ each represent hydrogen, $C_1$-$C_4$ alkyl or phenyl; and when $R_1$ and $R_2$ are not the same, the cis- or trans- isomers or mixtures thereof or the optical isomers (cis- or trans- or mixtures thereof).

It is obvious that in the case of formula (VI) and (VIII) that when either $R_{11}$ or $R_{12}$ is other than hydrogen, additional optical and cis- and trans-isomers are possible, and all of these are considered to be within the scope of EP-A-133 309.

Although dihydroimidazoisoindolediones are described as herbicidal agents in US-A-4,041,045 issued August 9, 1977, said publication does not render the compounds of this invention obvious since it does not disclose or imply the disclosure of herbicidal compounds containing a pyridine or substituted pyridine ring. Moreover, it is surprising to find that the formula I compounds of this invention are frequently found to be highly selective herbicidal agents effective for the preemergence and postemergence control of a wide variety of undesirable broadleaf weeds and grasses in the presence of graminaceous crops such as corn, rice and wheat; leguminous crops such as soybeans and in the presence of a variety of other crops including cotton.

In accordance with the process of EP-A-133 309, the formula (I) dihydroimidazopyrrolopyridines, quinolines and the like can be prepared by reaction of the appropriately substituted or unsubstituted formula (XII) 2-(2-imidazolidinyl)nicotinic acid; 2-(2-imidazolidinyl)quinoline-3-carboxylic acid; 2-(2-imidazclidinyl)-thieno- or furo[3,2-b]pyridine-6-carboxylic acid; 2-(2-imidazolidinyl)dihydrothieno- or furo[3,2-b]pyridine-6-carboxylic acid; 2-(2-imidazolidinyl)thieno- or furo[2,3-b]pyridine-5-carboxylic acid or 2-(2-imidazolidinyl)-dihydrothieno or furo[2,3-b]pyridine-5-carboxylicacid, with at least one molar equivalent and preferably a slight excess of acetic anhydride in the presence of pyridine and acetonitrile. In practice, it is generally desirable to warm the reaction mixture to 40 to 60 ° C until an essentially clear solution is obtained. Cooling of the thus-prepared solution to about ambient temperature or below then yields the desired formula (I) dihydroimidazopyrrolopyridine, quinoline, thieno or furo-pyridine or dihydrothieno or furopyridine, corresponding to the isomeric mixture of the substituted or unsubstituted acid employed as starting material for the reaction.

The reaction may be graphically illustrated as follows:

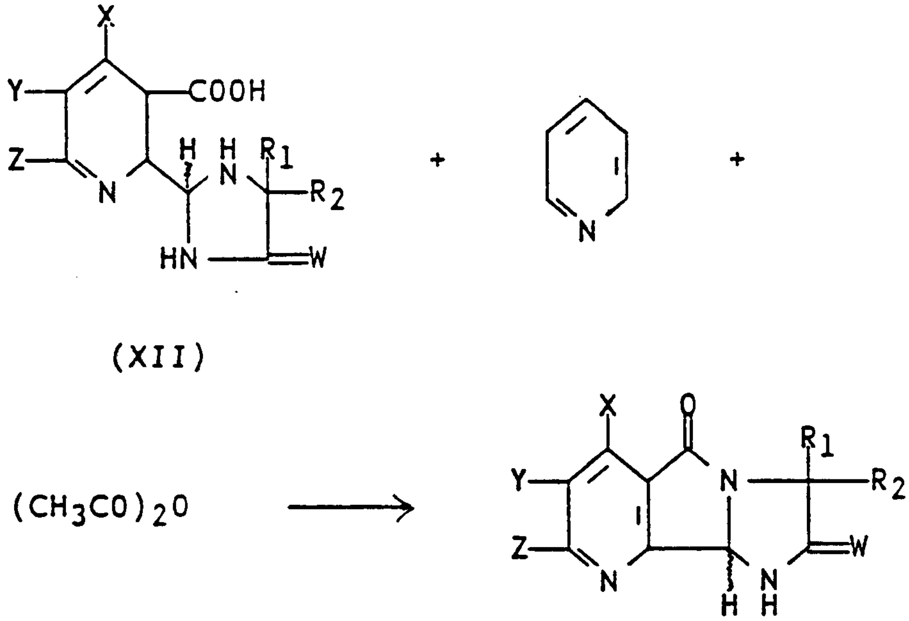


(XII)

(I)

wherein X, Y, Z, $R_1$, $R_2$ and W are as described for formula (I) above.

Similarly, in an alternate procedure it has also been found that the substituted or unsubstituted 2-(2-imidazolidinyl)nicotinic acid; 2-(2-imidazolidinyl)quinoline-3-carboxylic acid; 2-(2-imidazolidinyl)-thieno- or furo[3,2-b]pyridine-6-carboxylic acid; 2-(2-imidazolidinyl)dihydrothieno- or furo[3,2-b]pyridine-6-carboxylic acid; 2-(2-imidazolidinyl)thieno- or furo-[2,3-b]pyridine-5-carboxylic acid or 2-(2-imidazolidinyl)dihydrothieno- or furo[2,3-b]pyridine-5-carboxylic acid, which are themselves pre- and postemergence herbicides, can be converted to the corresponding formula (I) dihydroimidazopyrrolopyridine, quinoline, thieno- or furo[3,2-b]-pyridine, dihydrothieno- or furo-[3,2-b]pyridine, thieno- or furo[2,3-b]pyridine or the dihydrothieno- or furo-[2,3-b]pyridine, by reaction thereof with at least an equimolar amount of N,N′-dicyclohexylcarbodiimide in the presence of a chlorinated hydrocarbon solvent such as methylene chloride, dichloroethane or chloroform. The reaction may be conducted at ambient temperature and yields the desired formula (I) compound on evaporation or separation of the solvent from the reaction mixture. The reaction may be graphically illustrated as follows:

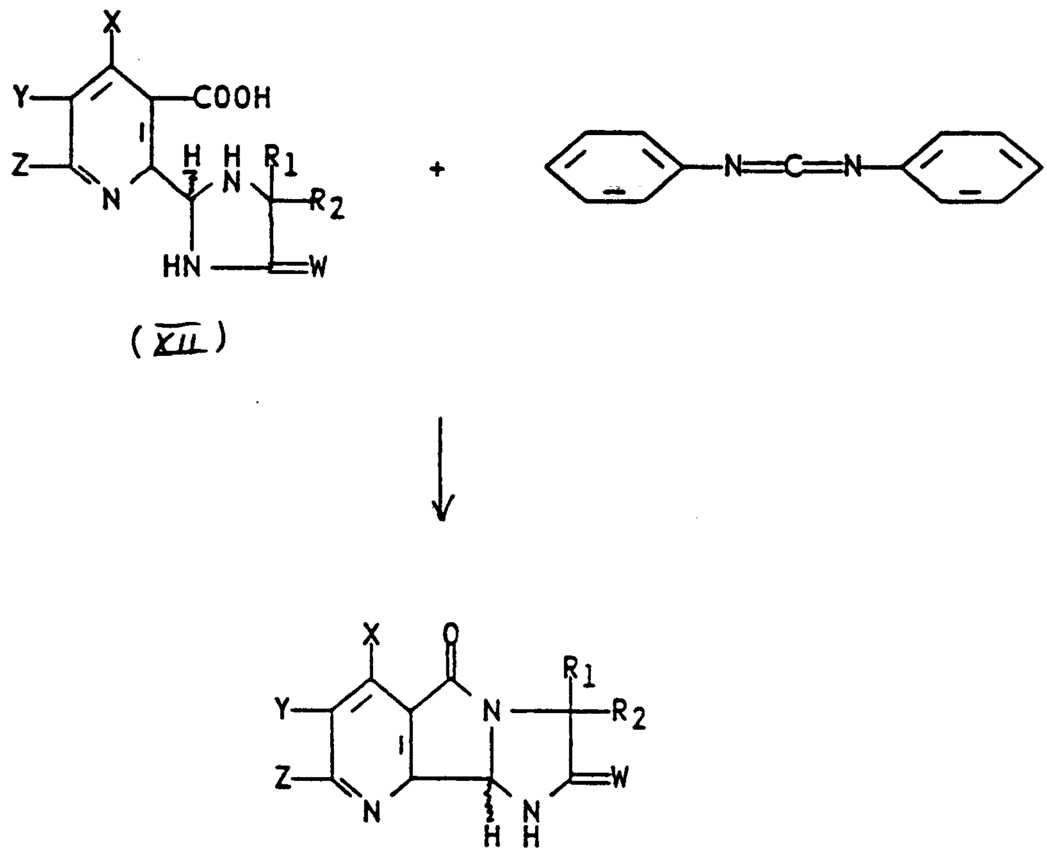

(XII)

(I)

wherein $R_1$, $R_2$, X, Y, Z and W are as described with respect to formula (I) above. It should be understood that if a cis-, trans- or mixture of cis- and trans-imidazolidinone is employed as starting material in the above reactions, then the cis-, trans- or mixture of cis- and trans- formula (I) compounds is obtained. These formula (I) dihydroimidazopyrrolopyridines, quinolines, thieno- and furopyridines and dihydrothieno- and furopyridines, are unexpectedly substantially more selective than their 2-(2-imidazolidinyl)acid precursors.

Many of the formula (I) dihydroimidazopyrrolopyridine, quinoline, thieno- and furopyridine and dihydrothieno- and furopyridine, compounds of EP-A-133 309, may also be prepared by reduction of the corresponding formula (XIV) 5H-imidazopyrrolopyridine, quinoline, thieno- or furopyridine, or dihydrothieno- or furopyridine, 2,5-dione. This reduction can be achieved by reaction of the formula (XIV) 2,5-dione with at least an equimolar amount of a reducing agent such as sodium or lithium borohydride in alcohol or aqueous alcohol or tetrahydrofuran or aqueous tetrahydrofuran, at a temperature between -10 and +5°C. Acidification of the reaction mixture to a pH of about 3, using a strong mineral acid such as concentrated sulfuric acid, then yields the desired formula (I) product. The reaction may be graphically illustrated as follows:

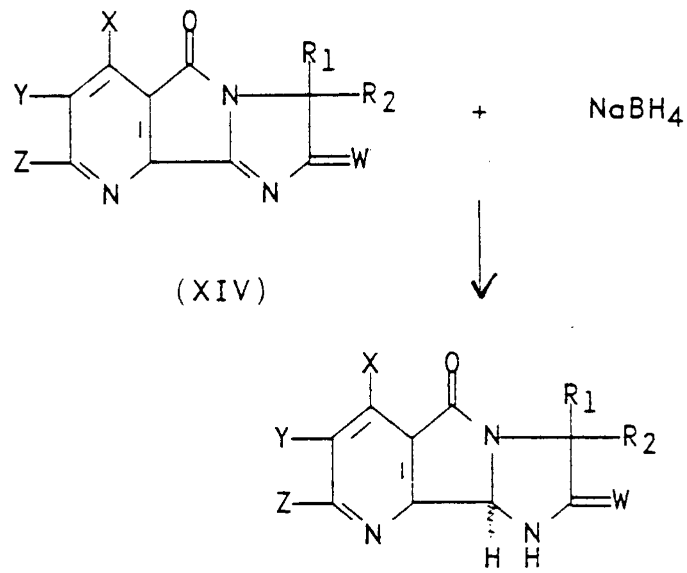


(XIV)

(I)

wherein X, Y, Z, W, $R_1$ and $R_2$ are as described with respect to formula (I) above.

This procedure usually forms a mixture of the cis and trans-isomers of the formula (I) compounds.

The preparation of the formula XIV 5H-imidazopyrrolopyridines and imidazopyrroloquinolines in which W is oxygen is described in the EPA 0,041,623, published December 16, 1981. The intermediates wherein W = S and their preparation are claimed in the present patent.

In all the cases described above, the products from the reaction are those in which A is hydrogen. In order to prepare these compounds in which A is a group other than hydrogen, advantage is taken of the ability of the $\rangle$C = N— function in the imidazopyraolopyridines, quinolines, thieno- and furopyridines to add a variety of nucleophiles such as alcohols, amines and thiols. The reaction may be graphically illustrated as follows:

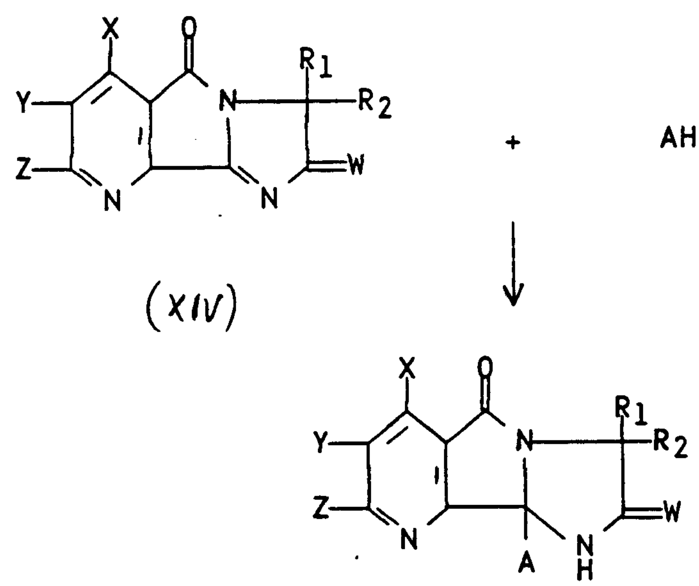


(XIV)

(I)

wherein AH is, for example, $CH_3OH$, $CH_3SH$, $CH_3NH_2$. This reaction may be catalyzed by acids such as p-toluenesulfonic acid or bases such as tertiary amines.

In practice it has been found that many substituted and unsubstituted aromatic and heteroaromatic imidazolidinone and imidazolidinethione compounds utilized as starting materials for the above described reactions by formula (I) can be prepared by reduction of the corresponding formula (XV) imidazolinone or imidazolinethione with, for example, at least about an equimolar amount of sodium cyanoborohydride in the presence of a solvent such as $C_1$-$C_4$ aliphatic alcohol, aqueous alcoholic mixture or ether, followed by acidification to a pH between 2.5 and 5 and preferably between 3 and 4, with a strong mineral acid such as hydrochloric acid, or an organic acid such as acetic or the like. This reduction is generally conducted at a temperature between 0 and 40°C and is particularly effective for treatment of 2-(2-imidazolinyl)nicotinic acids and esters, but preferably the methyl esters. It is likewise effective for reduction of the imidazolinyl function 2-(2-imidazolinyl)thieno and furo[3,2-b]-pyridine-6-carboxylic acid esters and 2-(2-imidazolinyl)-thieno and furo[2,3-b]pyridine-5-carboxylic acid esters.

The above described reduction may be graphically illustrated as follows:

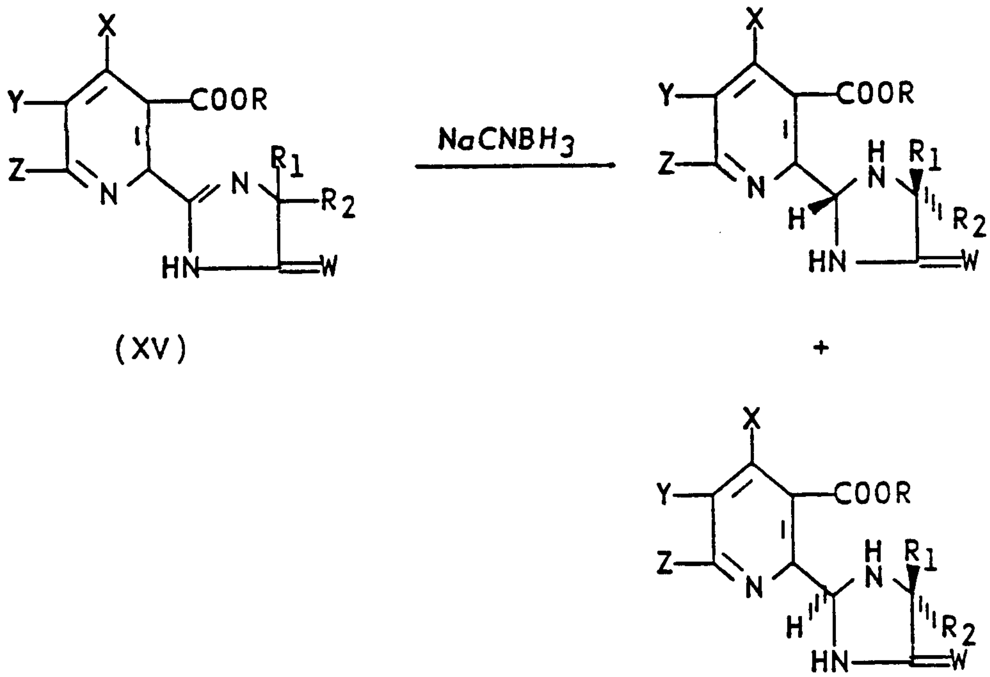


(XV)

Cis

Trans

wherein R may be hydrogen, but preferably a methyl group and $R_1$, $R_2$, W, X, Y and Z, are as defined in reference to said formula I. When R is a methyl group then this can advantageously be removed by reaction of the methyl ester in a $C_1$-$C_4$ aliphalic alcohol, preferably absolute methanol, and admixing therewith at least one equivalent of strong base.

As shown above, the imidazolidinones and imidazolidinethiones are obtained as a mixture of cis-and trans-isomers when $R_1$ and $R_2$ are not the same. These isomers are obtained in variable amounts. The mixtures are useful as such, but can frequently be separated chromatographically to give the pure cis- and trans-isomers, both of which are effective herbicidal agents.

Since the above-described reduction is not a universal method for the preparation of all substituted and unsubstituted aromatic and heteroaromatic imidazolidinones and imidazolidinethiones, a variety of synthetic routes have been explored in order to provide effective procedures for the manufacture of the imidazolidinones and imidazolidinethiones employed as starting materials for the preparation of the formula (I) compounds.

Accordingly, it has now been determined that both the oxo and thioxo derivatives of 2-(2-imidazolidinyl)-nicotinates and 2(2-imidazolidinyl)quinoline-3-carboxylates can be synthesized by heating to refluxing temperature, a mixture of a formula IX aminoamide or aminothioamide with about an equimolar amount of an appropriate formula X substituted or unsubstituted lower alkyl, 2-formylpyridine-3-carboxylate or 2-formylquinoline-3-carboxylate, in the presence of an inert organic solvent such as benzene, or toluene, and a strong organic acid, such as p-toluenesulfonic acid, under a blanket of nitrogen. The thus-formed ester

may then be converted to the corresponding formula (XII), acid, used as starting materials in the synthesis of the formula (I) compounds by dissolving or dispersing the imidazolidinone ester or imidazolidinethione ester in a $C_1$-$C_4$ aliphatic alcohol, preferably absolute methanol and admixing therewith at least one equivalent of strong base. In practice, the base is generally dissolved in water and the mixture heated to between 20 and 50° C. The mixture is then cooled and adjusted to pH 6.5 to 7.5, and preferably about pH 7, with a strong mineral acid such as hydrochloric acid to yield the imidazolidinone or imidazolidinethione acid wherein $R_1$, $R_2$, $R_3$, W, X, Y and Z are as defined above. The reaction is illustrated in Flow Diagram (I).

FLOW DIAGRAM I

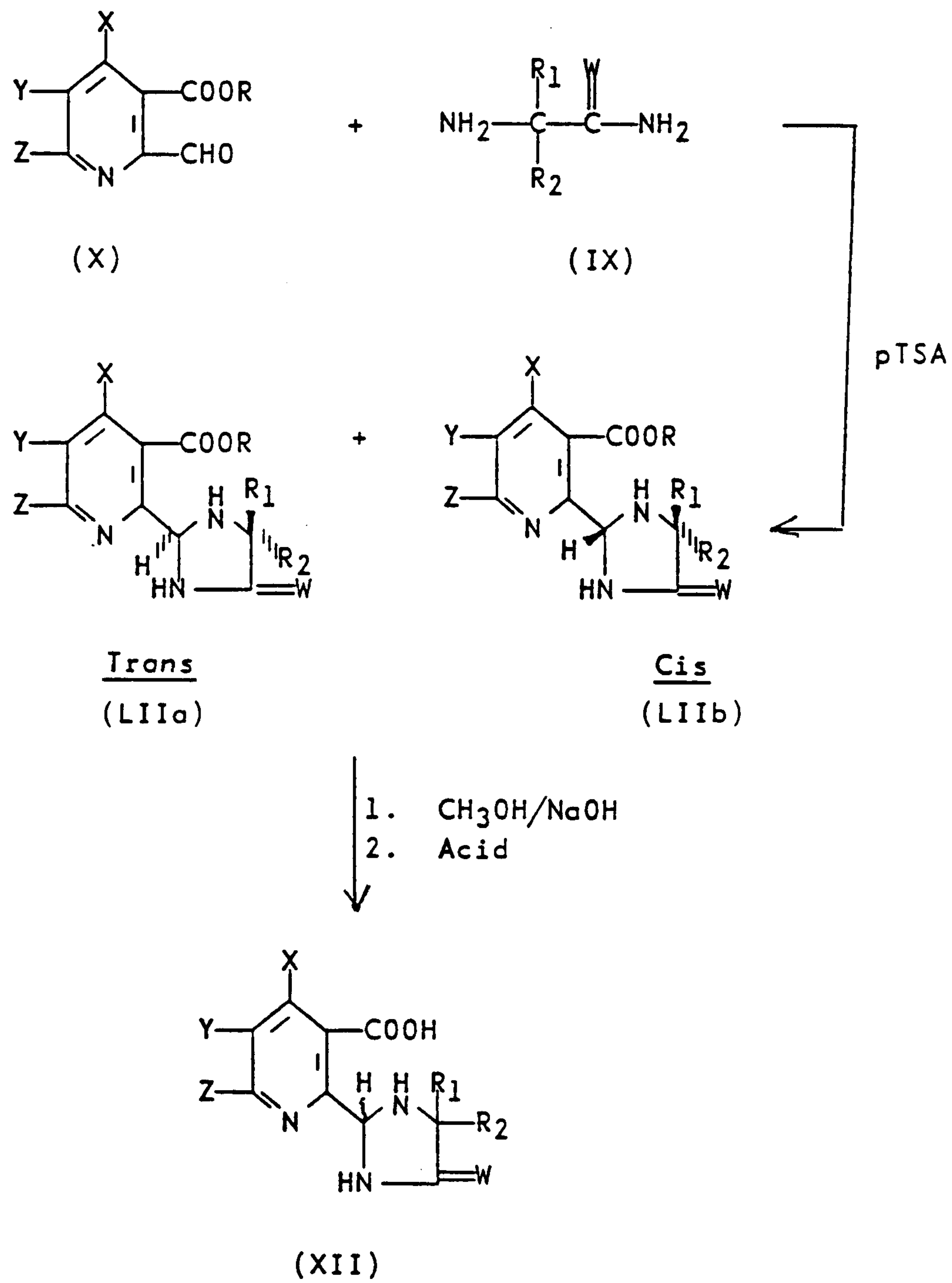


(X) + (IX)

Trans (LIIa) + Cis (LIIb)

(XII)

wherein R is $C_1$-$C_4$ alkyl; and $R_1$, $R_2$, X, Y, Z and W are as described for formula (I) above.

The reaction of an aldehyde of formula (X) with an α-aminoamide or thioamide of formula (IX) under acid catalysis gives the corresponding Schiff's base as the initial product. Whether the Schiff's base of general formula

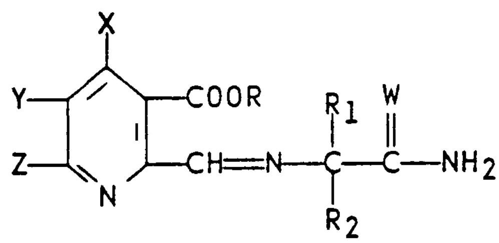

is isolated as such or cyclizes under the reaction conditions to the desired imidazolidinone depends on some unknown subtle factors. Nevertheless, if the Schiff's base is isolated it can, in a separate reaction, be cyclized with trifluoroacetic acid to the imidazolidinone.

Substituted $C_1$-$C_4$ alkyl 2-formylnicotinates which are useful in the preparation of 2-(2-imidazolidinyl)-nicotinatic acids and esters by the aldehyde route described above and illustrated in Flow Diagram I, for synthesis of formula LIIa and LIIb substituted 5-oxo-2-imidazolidinyl nicotinates and the formula (XII) acids, can be prepared from substituted $C_1$-$C_{12}$ alkyl 2-methylnicotinates. For convenience and clarity, the following synthesis is described using substituted methyl 2-methylnicotinates as illustrative of this class of reactions.

In accordance with the process, equivalent amounts of a substituted methyl 2-methylnicotinate, represented by formula LIII and m-chloroperbenzoic acid are admixed in the presence of a chlorinated hydrocarbon such as methylene chloride or chloroform. The reaction mixture is heated to refluxing temperature, then cooled to ambient temperature and excess peracid destroyed by addition of excess 1-hexene. Thereafter the solution is washed with sodium bicarbonate solution, dried and concentrated to give the corresponding substituted methyl methylnicotinate 1-oxide of formula LIV. The formula LIV 1-oxide is then heated to 70 to 95 °C with an excess of acetic anhydride to yield the formula LV substituted methyl 2-acetoxymethylnicotinate. A cosolvent such as pyridine or pyridine/dimethoxyethane may also be used in the reaction, but is not essential. Oxidation of the formula LV acetoxymethylnicotinate with hydrogen peroxide in acetic acid yields the methyl 2-acetoxymethylnicotinate 1-oxide represented by formula LVI. This 1-oxide is then readily converted to the formula LVII methyl 2-diacetoxymethylnicotinate by reaction with an excess of acetic anhydride at a temperature between 70 and 95 °C, with or without a cosolvent such as pyridine or pyridine/dimethoxyethane. Treatment of the formula LVII methyl diacetoxymethylnicotinate with an alkali metal alkoxide such as sodium methoxide, sodium ethoxide or potassium butoxide, in the presence of a $C_1$-$C_4$ aliphatic alcohol then yields the substituted alkyl formylnicotinate such as methyl 2-formylnicotinate of formula LVIII.

Alternatively, it has also been found that the reaction of a substituted $C_1$-$C_{12}$ alkyl 2-methylnicotinate, depicted by formula LIII, with benzaldehyde at an elevated temperature, yields the formula LIX methyl 2-styrylnicotinate which, when ozonized gives the formula LVIII substituted alkyl formylnicotinate.

Additionally, it has been found that treatment of the formula LV substituted methyl 2-acetoxymethylnicotinate with an alkali metal alkoxide such as sodium methoxide, in the presence of a lower aliphatic alcohol at an elevated temperature, yields the corresponding substituted methyl 2-hydroxymethylnicotinate of formula LX. The substituted methyl 2-hydroxymethylnicotinate is then converted to the formula LVIII substituted methyl formylnicotinate by oxidation with selenium dioxide or lead tetraacetate.

The above reactions are graphically illustrated in Flow Diagram II.

## FLOW DIAGRAM II

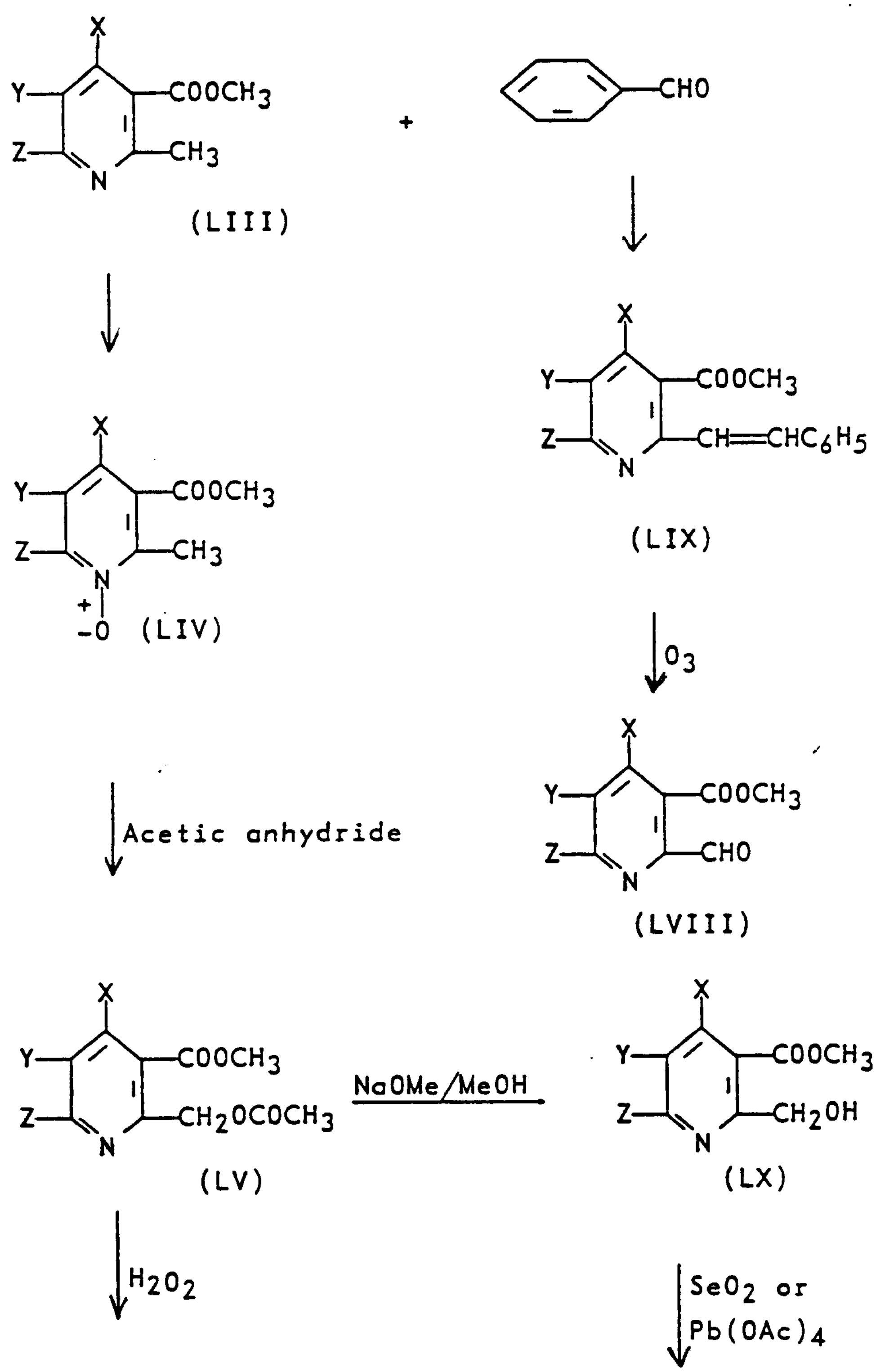


(LIII) (LIV) (LIX) (LVIII) (LV) (LX)

## FLOW DIAGRAM II (Continued)

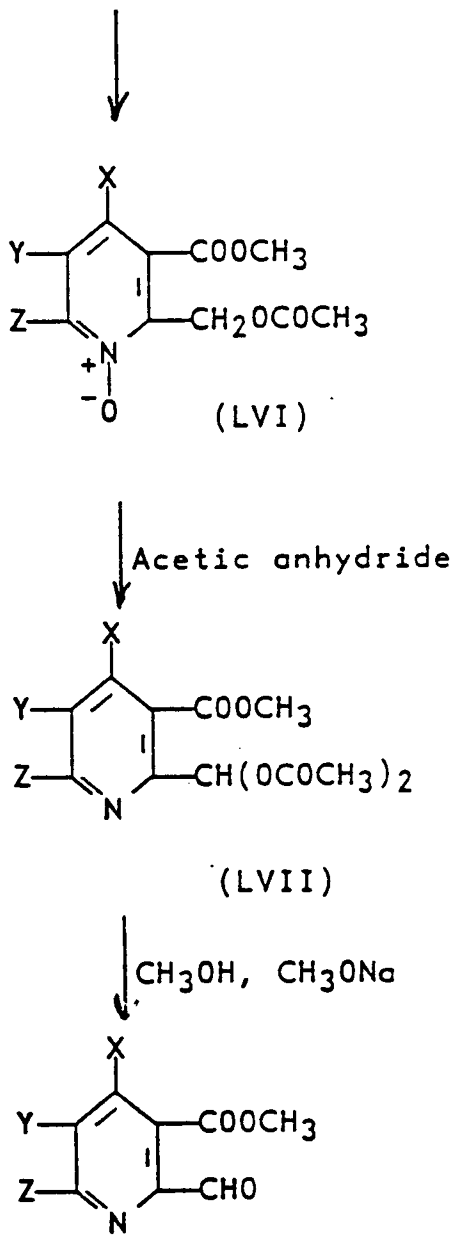


(LVI)

(LVII)

(LVIII)

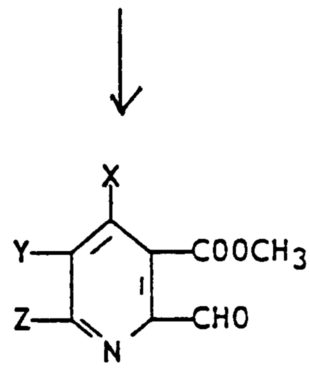

(LVIII)

The reduction of quinolinic acid diesters with diisobutylaluminum hydride is also an effective route to alkyl 3-formylnicotinates. The synthesis of these quinolinic acid diesters is described in EP-A-0 041 623.

The aldehyde route to the preparation of the formula LIIa and LIIb substituted (5-oxo(and thioxo)-2-imidazolidinyl)nicotinates is likewise effective for the preparation of the substituted and unsubstituted (5-oxo-2-imidazolidinyl)quinoline-3-carboxylates from the substituted 2-formylquinoline-3-carboxylates.

The process for the preparation of these substituted 2-formylquinoline-3-carboxylate intermediates involves the reaction of an appropriately substituted aniline, depicted by formula LXI:

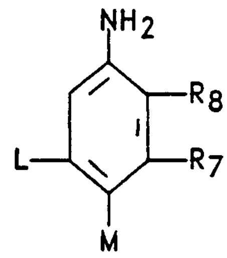

(LXI)

wherein L, M, $R_7$ and $R_8$ are as defined in reference to formula III quinolines; with approximately an equimolar amount of a keto-ester depicted by formula LXII and having the structure:

$R'—CO—CH_2COOR''$ (LXII)

wherein R′ is $CH_3$ or COOR″ and R″ is $C_1$-$C_4$ alkyl. This reaction is optionally conducted in the presence of an organic sulfonic acid such as p-toluenesulfonic acid hydrate, camphorsulfonic acid, or aniline hydrochloride, in the presence of an organic solvent such as cyclohexane, toluene, benzene, xylene, monochlorobenzene, orthodichlorobenzene and mixtures thereof, at a temperature from 20 to 110 ° C. It is preferred to continuously remove the water which is formed during the reaction by distillation either at atmospheric or under reduced pressures of as low as 50 mm of Hg while maintaining the reaction temperature in a range of 75 to 80 ° C. The reaction yields the $\beta$-anilino-$\alpha$,$\beta$-unsaturated ester of formula LXIII i.e.,

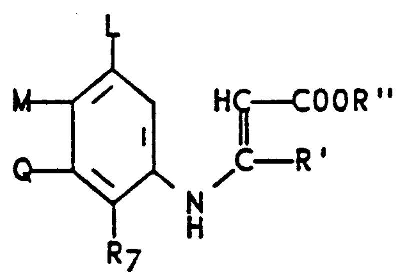

(LXIII)

wherein L, M, Q, $R_7$, R′, and R″ are as described above.

The thus-formed $\beta$-anilino-$\alpha$,$\beta$-unsaturatedester of formula LXIII is then reacted with an approximately equimolar amount of an immonium salt having the structure:

$$Cl—CH{=}\overset{\oplus}{N}(R''')_2\cdot Cl^{\ominus},$$

(LXIV)

wherein R‴ is $C_1$-$C_6$ alkyl or

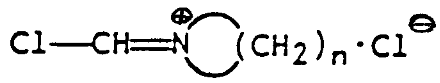

(LXIVa)

wherein n is 4 or 5, and referred to respectively as formula LXIV or LXIIa. The reaction is conducted in the presence of a hydrocarbon solvent such as toluene or a chlorinated hydrocarbon solvent such as methylene chloride, dichloroethane, orthodichlorobenzene, chlorobenzene, or mixtures thereof, at a temperature between 40 and 110°C, for a period of time sufficient to essentially complete the reaction and yield the formula LXV alkyl ester of 2-methyl-3-quinolinecarboxylic acid, if R′ is $CH_3$ in the formula LXVII β-anilino-α,β-unsaturated ester or the quinoline-2,3-dicarboxylate if R′ is COOR″ in the formula LXVIII β-anilino-α,β-unsaturated ester.

Alternatively, the formula LXI substituted aniline, wherein L, M, $R_7$ and $R_8$ are as described above, can be reacted with about an equimolar amount of a formula LXVI acetylene dicarboxylate having the structure:

R″OOC—C≡C—COOR″,

where R″ is $C_1$-$C_4$ alkyl. This reaction is generally carried out in the presence of a solvent such as dichloroethane or a $C_1$-$C_4$ alcohol such as methanol, at a temperature between 0 and 100°C to yield a β-anilino-α,β-unsaturated ester as formula LXIII. The β-anilino-α,β-unsaturated ester of formula LXIII is then reacted with an immonium salt depicted by formula LXIV having the structure:

$$Cl—CH{=}\overset{\oplus}{N}(R''')_2 \cdot Cl^{\ominus},$$

wherein R‴ is $C_1$-$C_6$ alkyl or LXIVa having the structure:

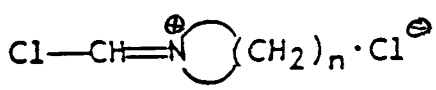

where n is 4 or 5. While the anion in formulas LXIV or LXIVa is shown as $Cl^{\ominus}$, it should be recognized that when $POCl_3$ is used to prepare the Vilsmeier reagent, the anion is

$$PO_2Cl_2^{\ominus}.$$

This reaction is generally conducted in the presence of a solvent such as methylene chloride, dichloroethane, monochlorobenzene, orthodichlorobenzene, or toluene at a temperature between 40 and 110°C for a period of time sufficient to complete the reaction and yield the quinoline-2,3-dicarboxylate shown as formula LXVa having the structure:

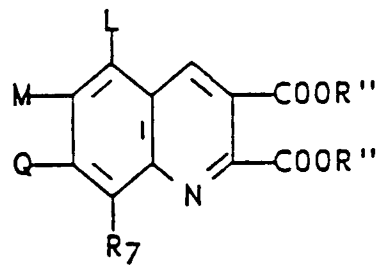

(LXVa)

wherein L, M, Q, $R_7$ and R″ are as described above.

The immonium salt formula LXIV or LXIVa utilized in the above cyclization reactions may, hereafter, be referred to as the Vilsmeier reagent. This reagent may be generated from a (N,N-dialkyl or N-alkyl,N-phenyl) formamide reaction with $POCl_3$, $COCl_2$, ClCO-COCl or $SOCl_2$ in a hydrocarbon or chlorinated hydrocarbon solvent.

Conversion of the 2-methyl-3-quinoline-carboxylate shown as formula LXV in which $R' = CH_3$ to the corresponding aldehyde of formula LXVII can be achieved in a manner similar to that described above for the conversion of the substituted 2-methylnicotinate of formula LIII to the corresponding 2-formylnicotinate of LVIII.

Conversion of the quinoline-2,3-dicarboxylate, shown as formulas LXV and LXVa, to the corresponding aldehyde shown as formula LXVII having the structure:

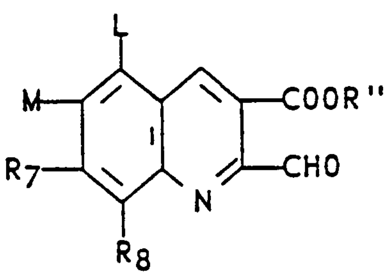

(LXVII)

where L, M, $R_7$ $R_8$ and R″ are as defined above, can be achieved by reaction of the formula LXV quinoline-2,3-dicarboxylate with diisobutylaluminum hydride. The reaction is preferably conducted in the presence of a non-protic solvent such as tetrahydrofuran under a blanket of inert gas.

These reactions are graphically illustrated in Flow Diagram III below.

## FLOW DIAGRAM III

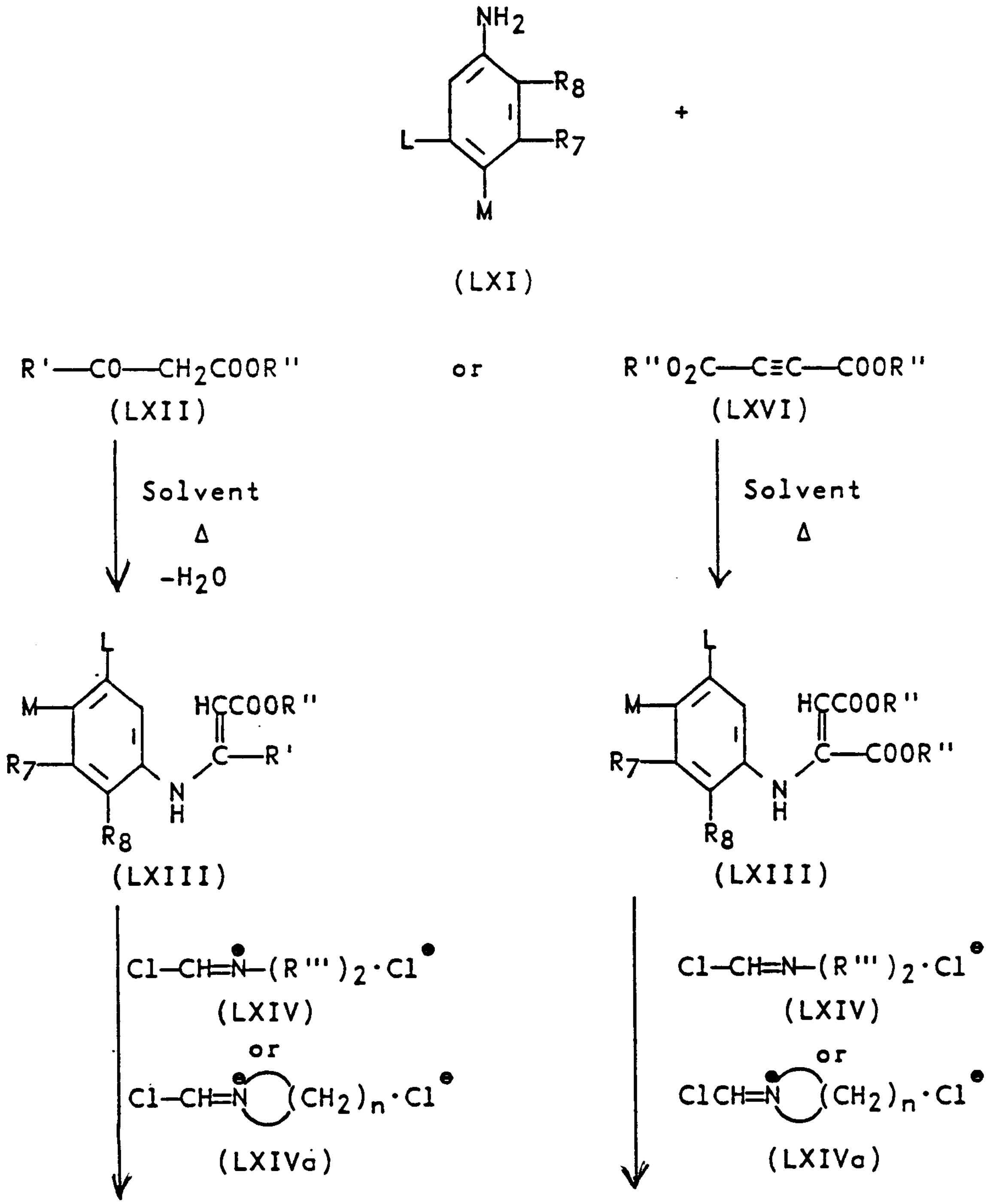


(LXI)

(LXII) or (LXVI)

(LXIII) (LXIII)

(LXIV) or (LXIVa)

(LXIV) or (LXIVa)

FLOW DIAGRAM III (Continued)

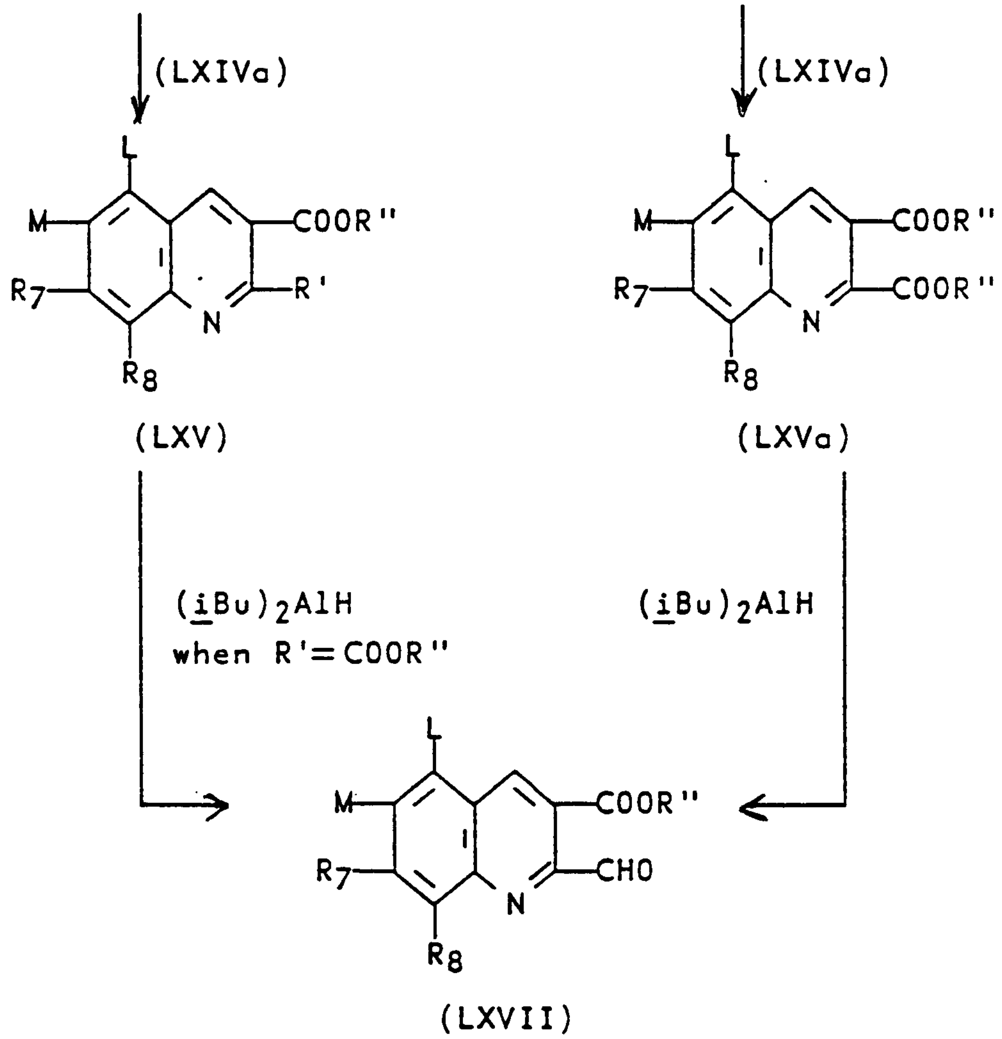


(LXV) (LXVa)

(LXVII)

The 2-(2-imidazolidinyl)thieno- and furo[3,2-b]pyridine-6-carboxylates; 2-(2-imidazolidinyl)-2,3-dihydrothieno- and furo[3,2-b]pyridine-6-carboxylates; 2-(2-imidazolidinyl)thieno- and furo[2,3-b]pyridine-5-carboxylates and 2-(2-imidazolidinyl)-2,3-dihydrothieno- and furo[2,3-b]pyridine-5-carboxylates, useful as intermediates in the preparation of the formula (I) compounds can be obtained, by reduction of the corresponding (2-imidazolin-2-yl)thieno- and furo[2,3-b] and [3,2-b]pyridines with sodium cyanoborohydride. These 2-(2-imidazolin-2-yl)thieno- and furo[2,3-b] and [3,2-b]pyridine intermediates, necessary for the preparation of the formula V, VI, VII and VIII, thieno- and furopyridines, are described in the copending application for United States Letters Patent of Marinus Los, David William Ladner and Barrington Cross, Serial No 500,219, filed June 2, 1983.

The 2-(2-imidazolin-2-yl)thieno and furo[2,3-b] and [3,2-b]pyridine intermediates, used in the synthesis of the 2-(2-imidazolidinyl)thieno- and furo[2,3-b] and [3,2-b]pyridines starting materials for the compounds of EP-A-133 309 are depicted by formulas Va, VIa, VIIa and VIIIa, illustrated below.

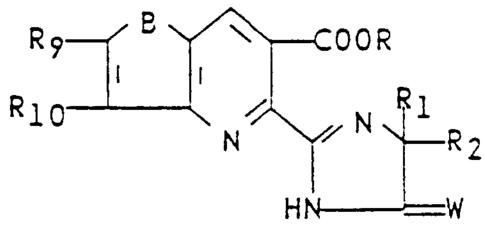

(Va)

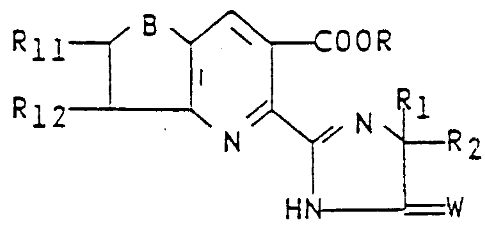

(VIa)

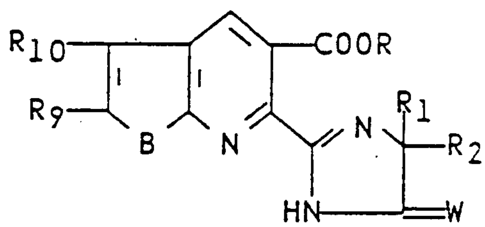

(VIIa)

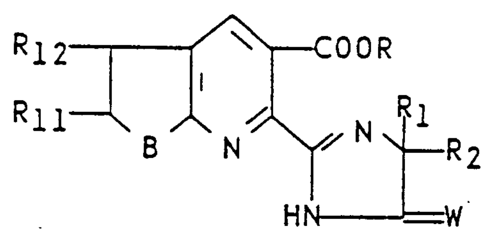

(VIIIa)

wherein R is hydrogen or $C_1$-$C_4$ alkyl and $R_1$, $R_2$, $R_9$, $R_{10}$, $R_{11}$, $R_{12}$, B and W are as described above in reference to compounds of formula V, VI, VII and VIII.

While for convenience, the imidazolinone and imidazolinethione intermediates referred to throughout are illustrated by single structures, it should be recognized that the imidazolinyl function in these compounds may exist in either tautomeric form, i.e:

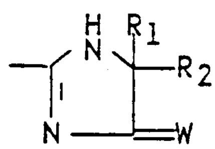

or

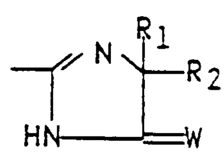

The formula Va, VIa, VIIa and VIIIa, intermediates for the compounds of EP-A-133 309 may be prepared from the appropriately substituted thieno- and furo[2,3-b] and [3,2-b]pyridinedicarboxylic acids and esters of formulas LXXI and LXXIa illustrated below.

Since $R_9$ and $R_{10}$ represent substituents selected from hydrogen, halogen, $C_1$-$C_4$ alkyl and phenyl, and $R_{11}$ and $R_{12}$ represent hydrogen, $C_1$-$C_4$ alkyl and phenyl; for the purposes of the following discussion, which relates to the preparation of the formula Va, VIa, VIIa and VIIIa, 2-(2-imidazolin-2-yl)thieno and furo-[2,3-b] and [3,2-b]pyridines, compound structures involved in the synthesis under discussion will be illustrated with $R_9$ and $R_{10}$.

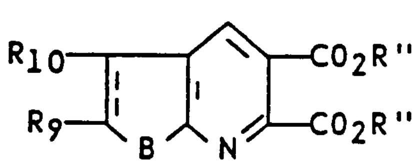

(LXXI)

and

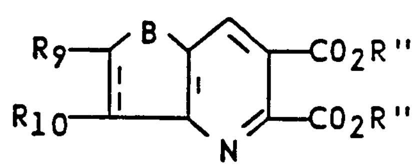

(LXXIa)

wherein $R_9$, $R_{10}$ and B are as previously described and R″ is methyl or ethyl.

Methods suitable for preparing formula Va, VIa, VIIa and VIIIa unsaturated compounds wherein - - is a double bond from the formula (LXXI) and (LXXIa) pyridinedicarboxylic acid esters are illustrated in Flow Diagram IV below.

Thus formula (LXXI) and (LXXIa) diesters may be hydrolyzed to the corresponding thieno- and furo-2,3-pyridinedicarboxylic acids of formula (LXXII) and (LXXIIa) by reaction thereof with a strong base such as potassium hydroxide or sodium hydroxide. Acid anhydrides of formula (LXXIII) and (LXXIIIa) may then be prepared by treatment of the formula (LXXII) and (LXXIIa) pyridinedicarboxylic acids with, for example, acetic anhydride. Reaction of formula (LXXIII) and (LXXXIIIa) anhydrides with an appropriately substituted aminocarboxamide or aminothiocarboxamide depicted by formula (IX) yields carbamoyl nicotinic acids of formula (LXXIV) and (LXXIVa). Treatment of the thus-formed formula (LXXIV) and (LXXIVa) carbamoyl nicotinic acids with about 2 to 10 molar equivalents of aqueous or aqueous alcoholic sodium or potassium hydroxide, preferably under a blanket of inert gas such as nitrogen, cooling and acidifying to pH 2 to 4 with a strong mineral acid such as hydrochloric acid or sulfuric acid gives herbicidally effective 6-(4,4-disubstituted-5-oxo-(or thiono)-2-imidazolin- 2-yl)thieno-and furo[2,3-b]pyridine-5-carboxylic acids, and 5-(4,4-disubstituted-5-oxo-(or thiono)-2-imidazolin-2-yl)thieno-and furo[3,2-b]pyridine-6-carboxylic acids encompassed by formulas (Va) and (VIIa).

Formula (Va) and (VIIa) 5-(2-imidazolin-2-yl)thieno- and furopyridine esters, wherein R represents a substituent other than hydrogen or a salt-forming cation, and $R_1$, $R_2$, $R_9$, $R_{10}$ and B are as described above can be prepared by reacting a novel thieno- or furoimidazopyrrolopyridinedione, represented by formulas (LXXV) and (LXXVa), hereinbelow, in Flow Diagram (V) with an appropriate alcohol and corresponding alkali metal alkoxide at a temperature ranging between 20 and 50 ° C.

Formula (LXXV) and (LXXVa) thieno- and furoimidazopyrrolopyridinediones may conveniently be prepared from formula (VIIa) and (Va) acids, where R is H by treatment with one equivalent of dicyclohexylcarbodiimide in an inert solvent such as methylene chloride as illustrated in Flow Diagram (V) below.

FLOW DIAGRAM (IV)

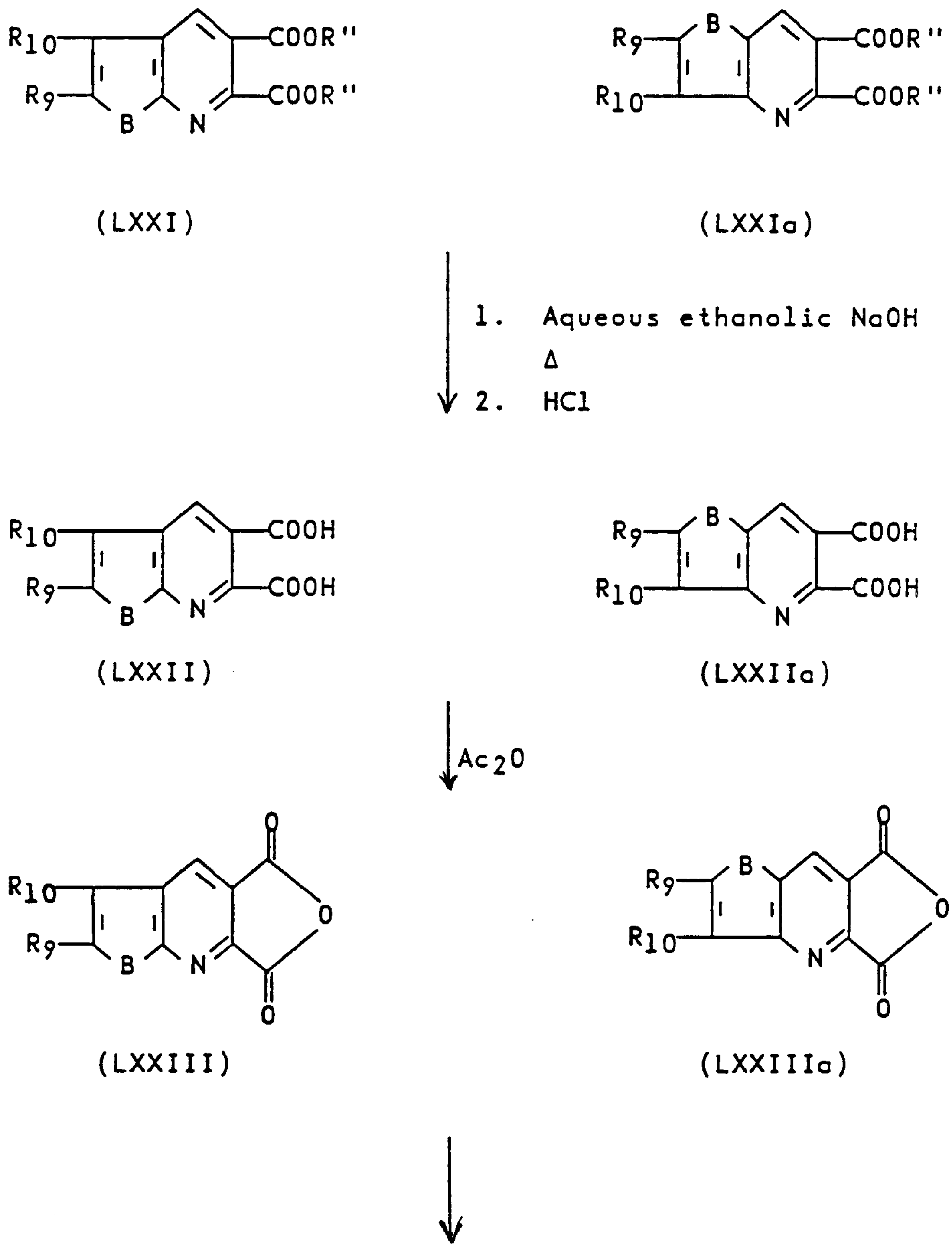


(LXXI) (LXXIa)

(LXXII) (LXXIIa)

(LXXIII) (LXXIIIa)

FLOW DIAGRAM (IV) (Continued)

(LXXIII) (LXXIIIa)

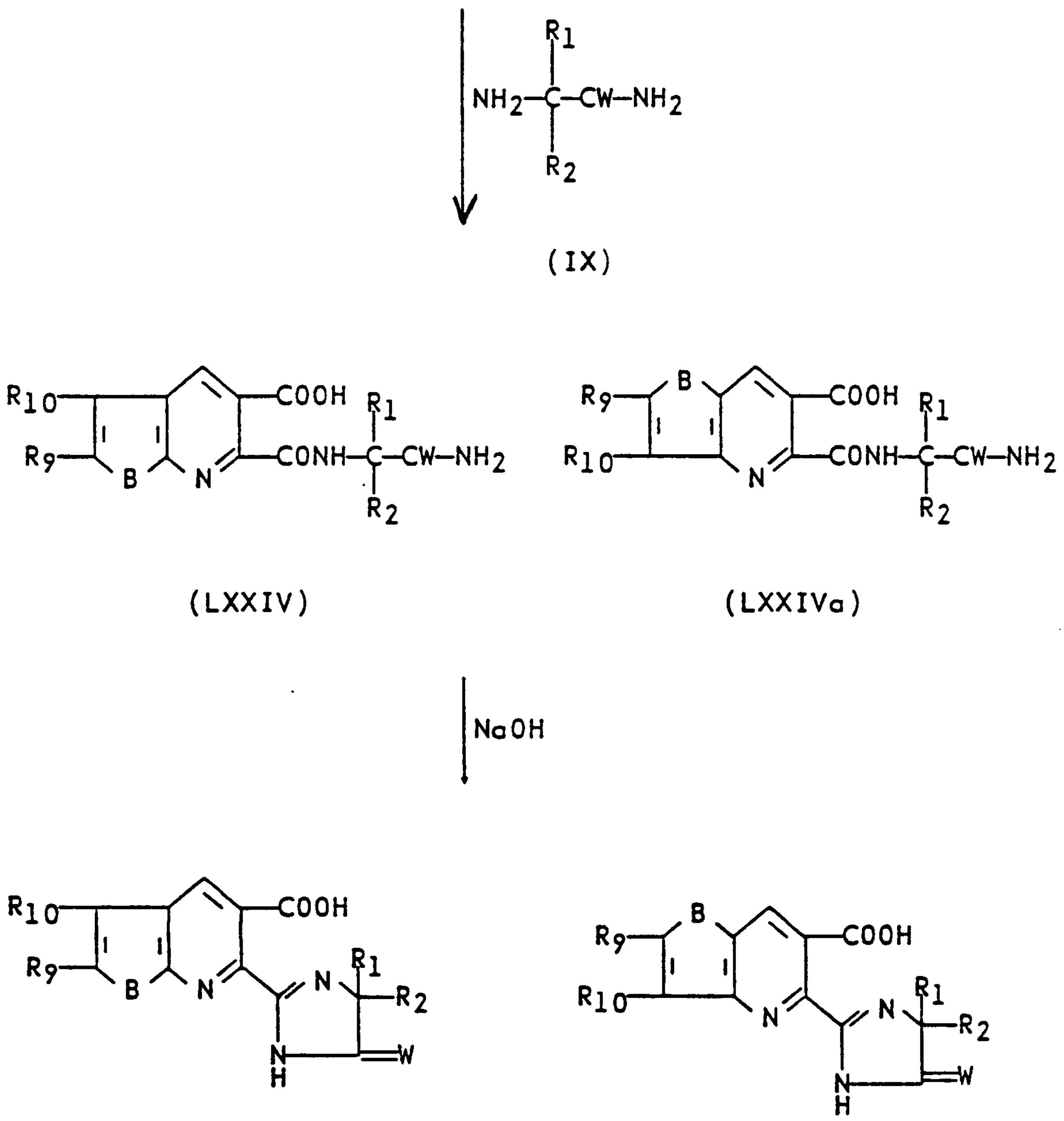


(IX)

(LXXIV) (LXXIVa)

FLOW DIAGRAM (V)

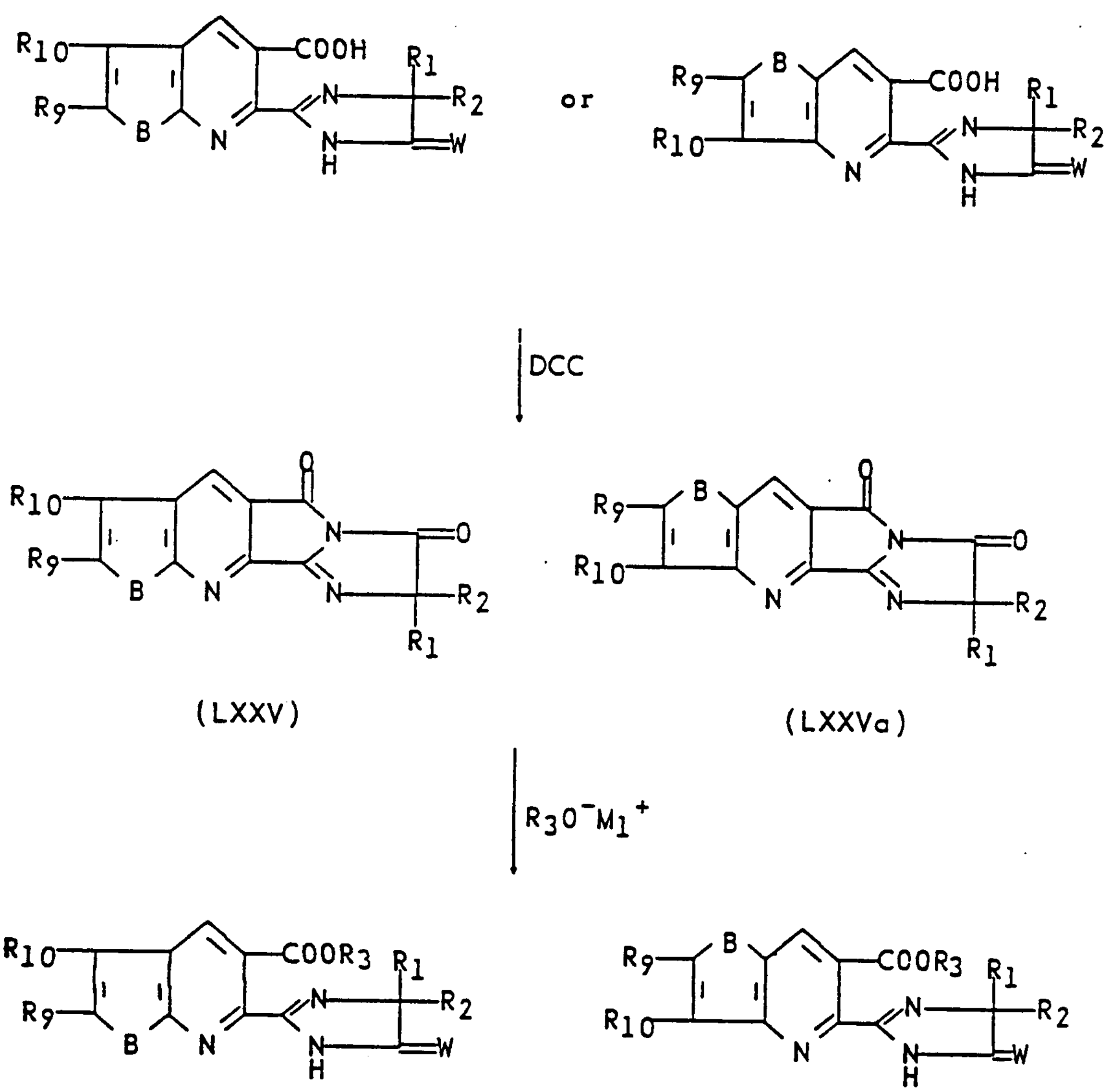


(LXXV)

(LXXVa)

where $M_1$ is an alkali metal, and X, Y, Z, $R_1$, $R_2$ are as above defined and $R_3$ is $C_1$-$C_4$ alkyl.

Many formula (LXXI) thieno[2,3-b]pyridinedicarboxylic acids and (LXXIa) thieno[3,2-b]-pyridinedicarboxylic acids may conveniently be prepared by reacting the appropriately substituted 2 or 3-aminothiophene of formula (LXXXIV) or (LXXXIVa) with a $C_1$-$C_4$ alkyl ester of acetylenedicarboxylic acid of formula (IX) as described by Bleckert et al. Chem. Ber. 1978, 106, 368. The thus-formed $\beta$-aminothieno-$\alpha,\beta$-unsaturated ester of formula (LXXXV) or (LXXXVa) is then reacted with an immonium salt depicted by the formula

$$Cl-CH=\overset{\oplus}{N}-(R''')_2\ \overset{\ominus}{Cl}$$

wherein $R'''$ is $C_1$-$C_6$ alkyl or

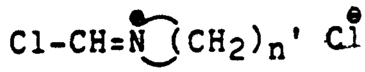

where n′ is 4 or 5, in the presence of a low boiling chlorinated hydrocarbon solvent such as methylene chloride or dichloroethane at a temperature between 40 and 90 °C, for a period of time sufficient to essentially complete the reaction and yield the formula (LXXI) [2,3-b]thieno- or (LXXIa) [3,2-b]thieno-2,3-pyridinedicarboxylic acid as the dialkyl ester as illustrated in Flow Diagram (VI) below.

The furo[3,2-b]pyridinecarboxylic acids may be prepared by reacting 3-amino-2-formylfuran of formula (LXXVI) prepared by the method of S. Gronowitz et al., Acta Chemica Scand B29 224(1975) with ethyl oxalacetate to give the furopyridine compounds directly, as illustrated in Flow Diagram (VII) below while the furo[2,3-b]pyridine compounds where $R_9$ and $R_{10}$ are H are obtained by bromination of the reaction product (LXXVII) of acetoacetamide with the diethyl ester of ethoxymethyleneoxalacetic acid followed by treatment with sodium borohydride and para-toluene sulfonic acid in refluxing xylene as illustrated in Flow Diagram (VIII) below.

# FLOW DIAGRAM (VI)

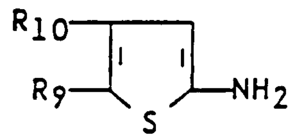

or

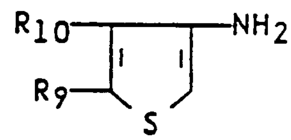

(LXXXIV) (LXXXIVa)

R''$O_2C-C\equiv C-COOR''$

(IX)

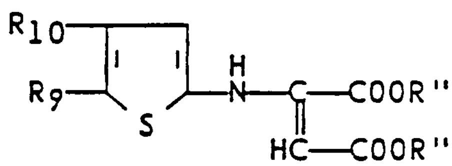

or

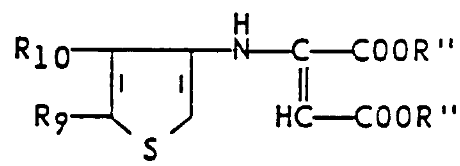

(LXXXV) (LXXXVa)

$Cl-CH=\overset{\oplus}{N}(R''')_2 \cdot Cl^{\ominus}$

or

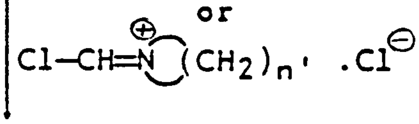

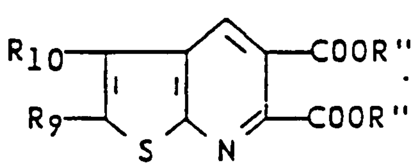

or

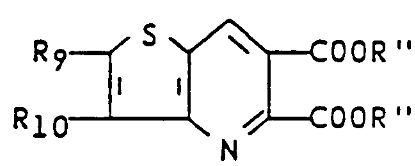

(LXXI) (LXXIa)

## FLOW DIAGRAM (VII)

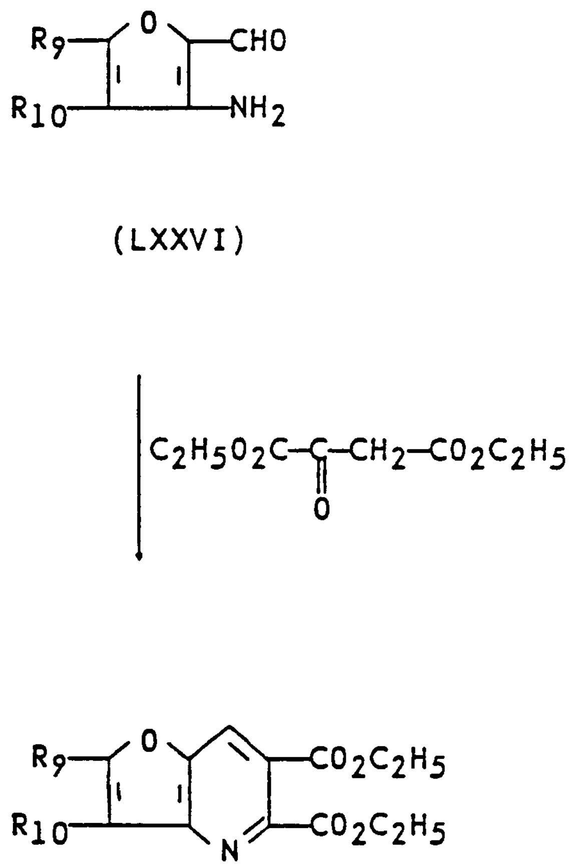

(LXXVI)

FLOW DIAGRAM (VIII)

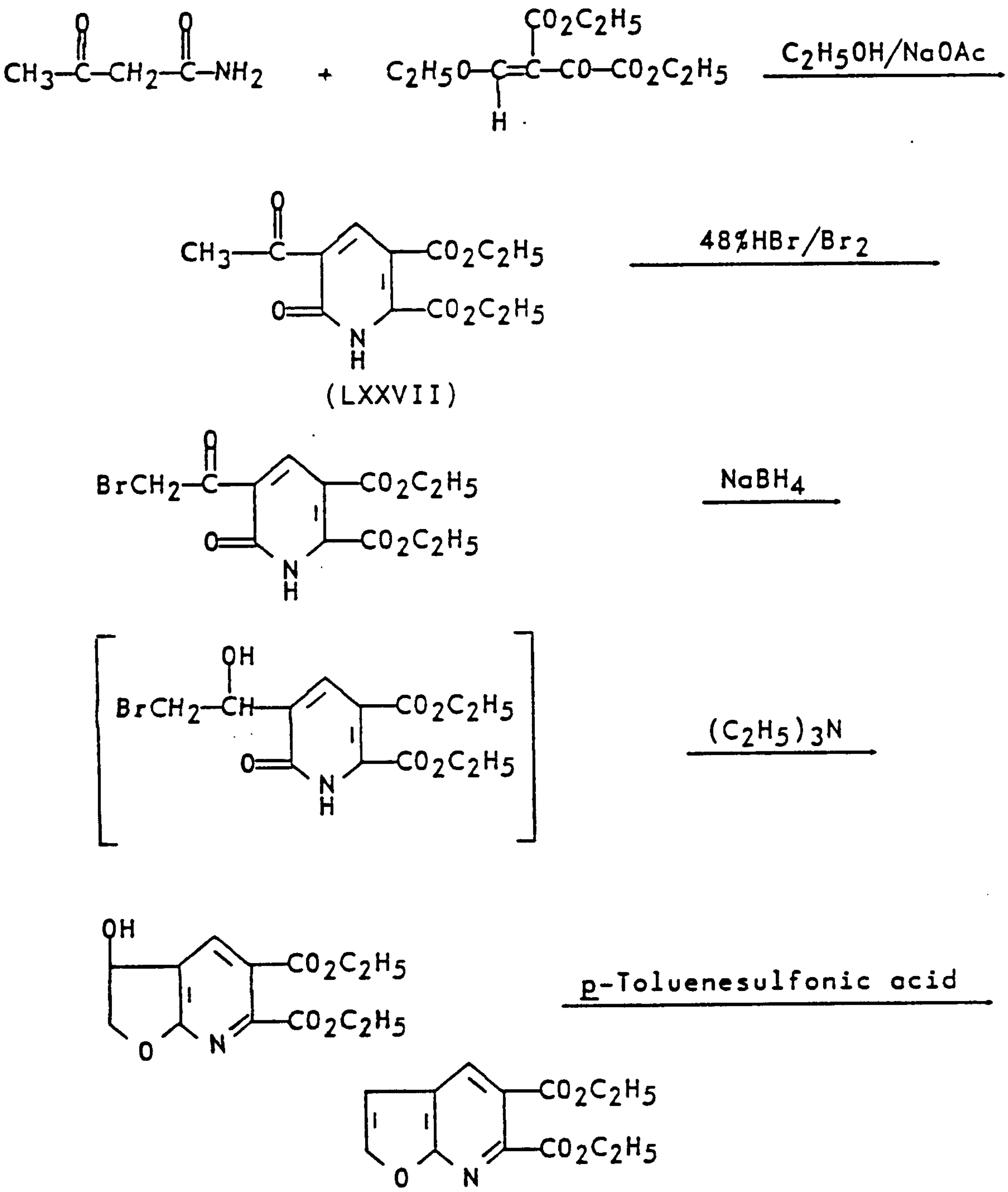


(LXXVII)

Substituents represented by $R_9$ and $R_{10}$ in formula (Va), (VIIa), (LXXV) and (LXXVa) compounds may be prepared either by using the appropriately substituted starting material for the preparation of formula (LXXI) and (LXXIa) thieno- and furopyridine-5,6-dicarboxylic acid esters or by electrophilic substitution (halogenation, nitration, sulfonation, etc.) directly upon formula (LXXI) or (LXXIa) diesters or Formula (Va) or (VIIa) final products, wherein at least one of Y or Z is hydrogen. These substituted formula (LXXI), (LXXIa) , (Va) and (VIIa) compounds then may be used as starting materials for additional $R_9$ and $R_{10}$ substitution by displacement, reduction, oxidation, etc. Representative substituted (LXXI) and (LXXIa) compounds which may be prepared by these procedures are as illustrated below.

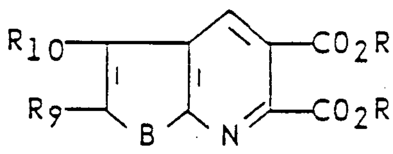

and

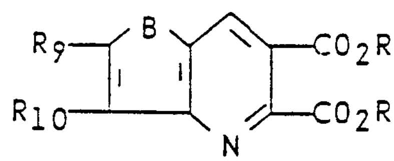

(LXXI) (LXXIa)

| B | $R_9$ | $R_{10}$ | R |
|---|---|---|---|
| S | H | H | $CH_3$ |
| S | H | Br | $CH_3$ |
| S | $CH_3$ | H | $CH_3$ |
| S | H | Cl | $CH_3$ |
| S | Cl | Cl | $CH_3$ |
| S | H | I | $CH_3$ |
| S | H | $NO_2$ | $CH_3$ |
| S | Br | Br | $CH_3$ |

| B | $R_9$ | $R_{10}$ | R |
|---|---|---|---|
| S | $CH_3$ | Cl | $CH_3$ |
| S | H | $CH_3$ | $CH_3$ |
| S | Cl | H | $CH_3$ |
| S | $CH_3$ | $CH_3$ | $CH_3$ |
| S | H | CN | $CH_3$ |
| S | H | $OCH_3$ | $CH_3$ |
| S | H | $N(CH_3)_2$ | $CH_3$ |
| S | H | $SCH_3$ | $CH_3$ |
| S | H | $OCF_2H$ | $CH_3$ |
| O | H | H | $C_2H_5$ |
| O | H | Br | $CH_3$ |
| O | H | Cl | $CH_3$ |
| O | $CH_3$ | H | $CH_3$ |
| O | $CH_3$ | H | $C_2H_5$ |
| O | H | $CH_3$ | $CH_3$ |
| O | $C_2H_5$ | H | $CH_3$ |
| O | H | $C_2H_5$ | $CH_3$ |
| O | $CH_3$ | $CH_3$ | $CH_3$ |
| S | $-(CH_2)_3-$ | | $CH_3$ |
| S | $-(CH_2)_4-$ | | $CH_3$ |
| S | $-(CH)_4-$ | | $CH_3$ |
| S | $C_6H_5$ | H | $CH_3$ |
| O | $C_6H_5$ | H | $CH_3$ |
| S | H | 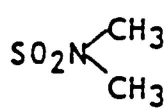 | $CH_3$ |
| S | H | $OC_6H_5$ | $CH_3$ |
| O | H | $OC_6H_5$ | $CH_3$ |
| O | $CF_3$ | H | $CH_3$ |

Additionally, novel herbicidal 2,3-dihydrothieno[2,3-b] and [3,2-b]pyridine compounds may be obtained by starting the sequence in Flow Diagram (VI) above with a dihydrothiophenimine hydrochloride. Novel herbicidal 2,3-dihydro furo[2,3-b] and [3,2-b]pyridines may be prepared by catalytic reduction of the formula (Va) or (VIIa) (2-imidazolin-2-yl) product, or (LXXI) and (LXXIa) furo[2,3-b] and [3,2-b]pyridine-5,6-diesters as for example with hydrogen and palladium on carbon, provided that $R_9$ and $R_{10}$ are substituents which are not reduced by such a procedure. This then provides novel 2,3-dihydro herbicidal compounds illustrated below.

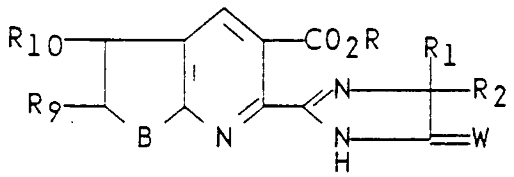

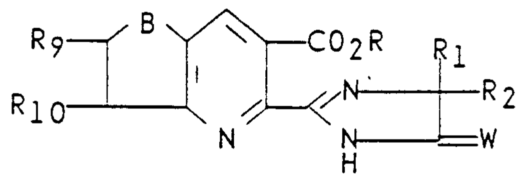

wherein $R_9$, $R_{10}$, B, W, $R_1$, $R_2$ and $R_B$ are as described for (Va) and (VIIA).

EXAMPLE 1

**Preparation of 2-isopropyl-2-methyl-5H-imidazo[1',2':1,2]pyrrolo[3,4-b]pyridine-3(2H),5-dione** - (Reference)

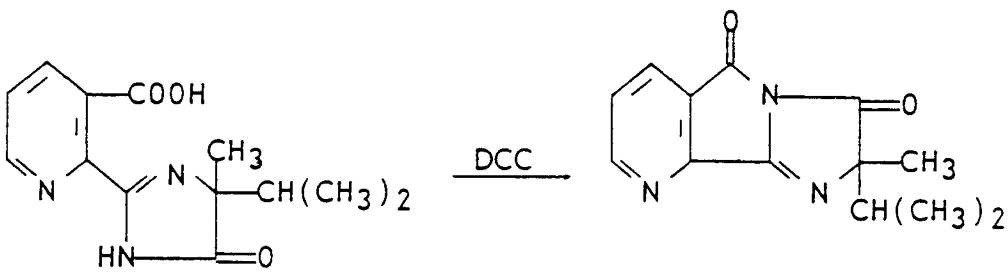


To a solution containing 50.9 g of dicyclohexylcarbodiimide in 600 mL of dry methylene chloride is added, while stirring, 60 g of the acid at such a rate that the temperature does not exceed 32° C. After stirring at room temperature for two and one-half hours, the mixture is filtered and the filtrate concentrated to give a white solid. This solid is recrystallized from methylene chloride to give 57.4 g of the dione, mp 125-128.5° C. The analytically pure dione melts at 132-134° C. The procedure is illustrated in European Patent Application 0,041,023, published December 16, 1981.

Using essentially the same procedure but substituting the appropriate acid for 2-(4-isopropyl-4-methyl-5-thione-2-imidazolin-2-yl)nicotinic acid, the following imidazopyrrolopyridine-3-thione-5-ones are prepared.

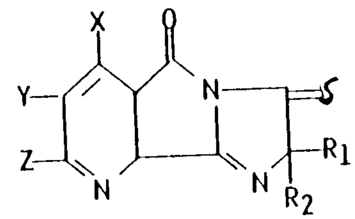

| W | X | Y | Z | $R_1$ | $R_2$ | mp°C |
|---|---|---|---|---|---|---|
| | H | H | H | $CH_3$ | $CH(CH_3)_2$ | 206.0 - 209.0 |
| | H | $CH_3$ | H | $CH_3$ | $CH(CH_3)_2$ | |
| | H | $C_2H_5$ | H | $CH_3$ | $CH(CH_3)_2$ | |
| | H | -CH=CH-CH=CH- | | $CH_3$ | $CH(CH_3)_2$ | 211.0 - 213.0 |

| X | Y | Z | $R_1$ | $R_2$ | mp°C |
|---|---|---|---|---|---|
| H | -CH=CH-S- | | $CH_3$ | $CH(CH_3)_2$ | |
| H | -CH=CH-O- | | $CH_3$ | $CH(CH_3)_2$ | |



EXAMPLE 2-

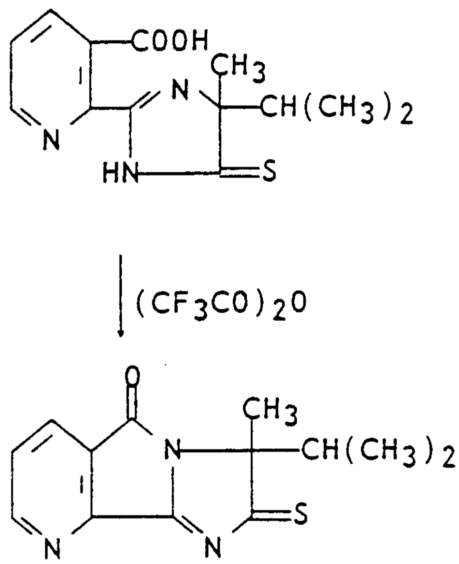


To a solution of the acid in 200 mL toluene and 100 mL $CH_2Cl_2$ under nitrogen is added with stirring at -60 ° C during 0.5 hours, 1.9 g trifluoroacetic anhydride dissolved in 18 mL toluene and 7 mL $CH_2Cl_2$. After this reaction, 500 mL hexane is added and the mixture concentrated in vacuo to give a dark green solid. NMR spectroscopy showed this to consist of 80% of the desired 2-thione-5-one and 20% of 3-thione-5-one.

The 2-thione-5-one was characterized as its water addition product, mp 162-165 ° C with structure

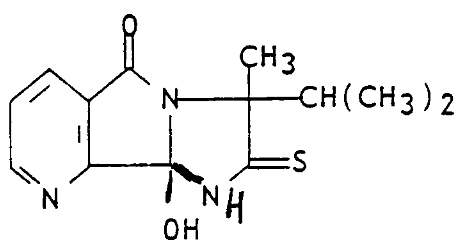

Using essentially this procedure or those described in Examples 1, 2 and 2-A and starting with the appropriate acid or 3-thione-5-one, the following 2-thiones-5-ones are obtained.

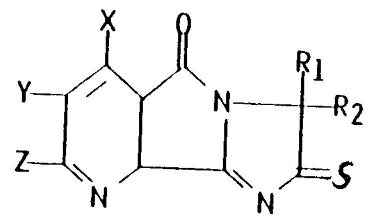

| X | Y | Z | $R_1$ | $R_2$ | mp°C |
|---|---|---|---|---|---|
| H | H | H | $CH_3$ | $CH(CH_3)_2$ | |
| H | $CH_3$ | H | $CH_3$ | $CH(CH_3)_2$ | |
| H | $C_2H_5$ | H | $CH_3$ | $CH(CH_3)_2$ | |
| H | -CH=CH-CH=CH- | | $CH_3$ | $CH(CH_3)_2$ | |

| X | Y | Z | R1 | R2 |
|---|---|---|---|---|
| H | | -CH=CH-S- | $CH_3$ | $CH(CH_3)_2$ |
| H | | -CH=CH-O- | $CH_3$ | $CH(CH_3)_2$ |

The procedures of Example 2 may be graphically illustrated as follows:

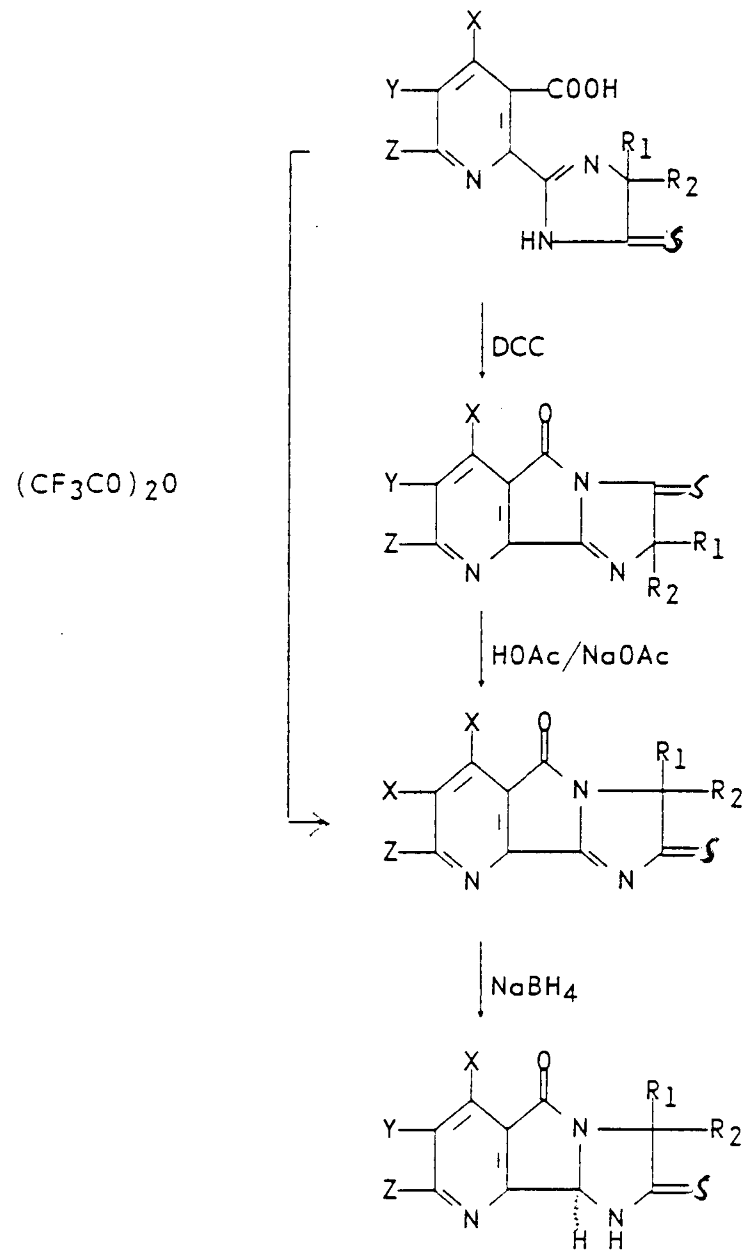


## Claims

1. A compound having the structure

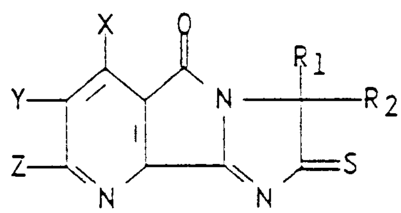

wherein

$R_1$ and $R_2$ each represent $C_1$-$C_3$ alkyl or cyclopropyl, with the proviso that the sum of the number of carbon atoms in $R_1$ and $R_2$ is 2 to 5; and when $R_1$ and $R_2$ are taken together with the carbon to which

they are attached, they may form a $C_3$-$C_6$ cycloalkyl ring optionally substituted with methyl;
X is hydrogen, halogen or methyl;
Y and Z are each hydrogen, halogen, $C_1$-$C_6$ alkyl, $C_1$-$C_4$ hydroxyalkyl, $C_1$-$C_6$ alkoxy, $C_1$-$C_4$ alkylthio, phenoxy, $C_1$-$C_4$ haloalkyl, $OCF_2CHF_2$,$OCF_3$, $OCHF_2$, nitro, cyano, $NR_4R_5$, $C_3$-$C_8$ straight or branched alkenyloxy optionally substituted with one to three halogens, $C_3$-$C_8$ straight or branched alkynyloxy optionally substituted with one to three halogens, or phenyl optionally substituted with one $C_1$-$C_4$ alkyl, $C_1$-$C_4$ alkoxy or halogen;
$R_4$ is hydrogen or $C_1$-$C_4$ alkyl;
$R_5$ is $C_1$-$C_4$ alkyl;
And, when taken together, Y and Z may form a ring in which YZ is represented by

(1) the structure: $-(CH_2)n-$, where n is an integer of 2, 3 or 4, provided that X is hydrogen; or

(2) by the structure:

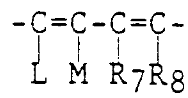

where, L, M, $R_7$ and $R_8$ each represent hydrogen, halogen, $C_1$-$C_4$ alkyl, $C_1$-$C_4$ alkoxy, $C_1$-$C_4$ alkylthio, $C_1$-$C_4$ alkylsulfonyl, $C_1$-$C_4$ haloalkyl, $NO_2$, CN, phenyl, phenoxy, amino, $OCF_3$, $OCHF_2$, $OCF_2CHF_2$, $C_1$-$C_4$ alkylamino, dialkyl($C_1$-$C_4$)amino, chlorophenyl, methylphenyl; $C_3$-$C_8$ straight or branched alkenyloxy optionally substituted with one to three halogens, $C_3$-$C_8$ straight or branched alkynyloxy optionally substituted with one to three halogens, or phenoxy substituted with one Cl $CF_3$, $NO_2$ or $CH_3$ group, with the proviso that only one of L, M, $R_7$ or $R_8$, may represent a substituent other than hydrogen, halogen, $C_1$-$C_4$ alkyl or $C_1$-$C_4$ alkoxy; or

(3) by the structures:

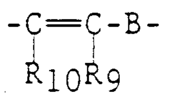 , 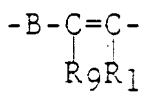 , 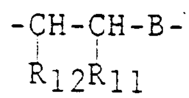 or 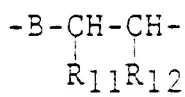 ;

where B is oxygen or sulfur; $R_9$ and $R_{10}$ each represent hydrogen, halogen, phenyl, or $C_1$-$C_4$ alkyl; $R_{11}$ and $R_{12}$ each represent hydrogen, $C_1$-$C_4$ alkyl or phenyl;

and when $R_1$ and $R_2$ are not the same, the optical isomers thereof.

**2.** A method for preparing a compound of claim 1 comprising reacting a compound having the structure

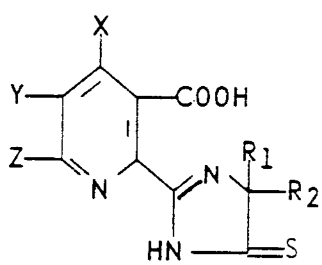

wherein $R_1$, $R_2$ X, Y and Z are as described in claim 1, with at least one molar equivalent of trifluroacetic anhydride.

**3.** A compound having the structure

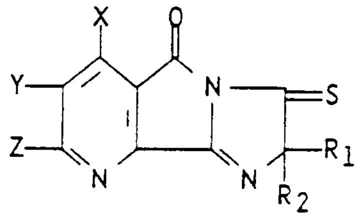

wherein
$R_1$, $R_2$, X, Y and Z as defined in claim 1.

**4.** A process for preparing a compound of claim 3 comprising reacting a compound having the structure

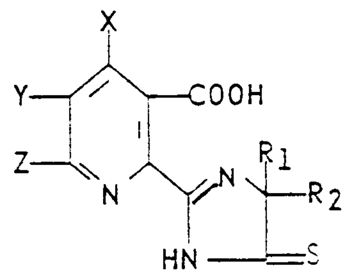

wherein $R_1$, $R_2$, X, Y and Z are as described in claim 3, with at least one molar equivalent of dicyclohexylcarbodiimide.

**5.** Use of a compound as defined in claim 3 for preparing herbicidal compounds having the structure

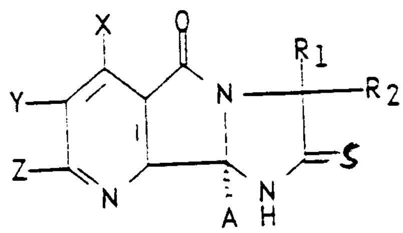

wherein $R_1$, $R_2$, X, Y and Z are as defined in said claim 3;
A is hydroxyl, $C_3$-$C_6$ alkenyloxy, $C_3$-$C_6$ alkynyloxy, $C_1$-$C_6$ alkylthio, $NR_{13}R_{14}$ or $C_1$-$C_6$ alkoxy optionally substituted with phenyl, halophenyl, $C_1$-$C_3$ alkylphenyl, $C_1$-$C_3$ alkoxyphenyl or di-$C_1$-$C_3$ alkylaminophenyl;
$R_{13}$ is hydrogen, $C_1$-$C_4$ alkyl optionally substituted with phenyl, halophenyl, $C_1$-$C_3$ alkylphenyl or $C_1$-$C_3$ alkoxyphenyl; and
$R_{14}$ is hydrogen or $C_1$-$C_4$ alkyl.

**Patentansprüche**

**1.** Verbindung mit der Struktur:

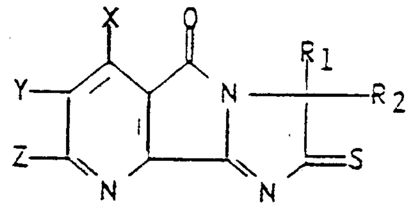

wobei

$R_1$ und $R_2$ jeweils für $C_{1-3}$-Alkyl oder Cyclopropyl steht, mit der Maßgabe, daß die Summe der Zahl der Kohlenstoffatome in $R_1$ und $R_2$ 2 bis 5 beträgt; und falls $R_1$ und $R_2$ zusammengefaßt sind mit dem Kohlenstoff, an das sie geknüpft sind, können sie einen $C_{3-6}$-Cycloalkylring bilden, der gegebenenfalls mit Methyl substituiert ist;

X für Wasserstoff, Halogen oder Methyl steht;

Y und Z jeweils für Wasserstoff, Halogen, $C_{1-6}$-Alkyl, $C_{1-4}$-Hydroxyalkyl, $C_{1-6}$-Alkoxy, $C_{1-4}$-Alkylthio, Phenoxy, $C_{1-4}$-Halogenalkyl, $OCF_2CHF_2$, $OCF_3$, $OCHF_2$, Nitro, Cyano, $NR_4R_5$, geradkettiges oder verzweigtes $C_{3-8}$-Alkenyloxy, das gegebenenfalls substituiert ist mit 1 bis 3 Halogen, geradkettiges oder verzweigtes $C_{3-8}$-Alkinyloxy, das gegebenenfalls substituiert ist mit 1 bis 3 Halogenen, oder Phenyl, das gegebenenfalls substituiert ist mit einem $C_{1-4}$-Alkyl, $C_{1-4}$-Alkoxy oder Halogen, steht;

$R_4$ für Wasserstoff oder $C_{1-4}$-Alkyl steht;

$R_5$ für $C_{1-4}$ Alkyl steht; und, falls sie zusammen gefaßt sind, können Y und Z einen Ring bilden, in dem YZ folgendes bedeutet:

(1) die Struktur $-(CH_2)_n-$, wobei n eine ganze Zahl von 2, 3 oder 4 ist, mit der Maßgabe, daß X für Wasserstoff steht; oder

(2) die Struktur :

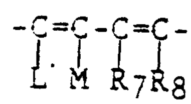

wobei L, M, $R_7$ und $R_8$ der jeweils für Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$ , CN, Phenyl, Phenoxy, Amino, $OCF_3$, $OCHF_2$, $OCF_2CHF_2$, $C_{1-4}$-Alkylamino, Dialkyl($C_{1-4}$)-Amino, Chlorphenyl, Methylphenyl, geradkettiges oder verzweigtes $C_{3-8}$-Alkenyloxy, gegebenenfalls substituiert mit 1 bis 3 Halogenen, geradkettiges oder verzweigtes Alkinyloxy, gegebenenfalls substituiert mit 1 bis 3 Halogenen oder Phenoxy, substituiert mit einer Cl, $CF_3$, $NO_2$ oder $CH_3$-Gruppe, steht, mit der Maßgabe, daß nur eines von L, M, $R_7$ oder $R_8$ einen Substituenten darstellen kann, der nicht Wasserstoff, Halogen, $C_{1-4}$ Alkyl oder $C_{1-4}$-Alkoxy ist; oder

(3) die Strukturen

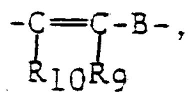 , 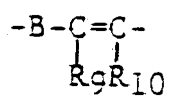 , 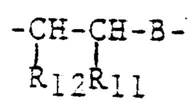 oder 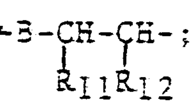 ;

wobei B für Sauerstoff oder Schwefel steht; $R_9$ und $R_{10}$ jeweils Wasserstoff, Halogen, Phenyl, oder $C_{1-4}$-Alkyl bedeuten; $R_{11}$ und $R_{12}$ jeweils Wasserstoff, $C_{1-4}$-Alkyl oder Phenyl bedeuten;

sowie, falls $R_1$ und $R_2$ nicht gleich sind, die optischen Isomeren derselben.

**2.** Verfahren zur Herstellung einer Verbindung gemäß Anspruch 1, umfassend die Umsetzung einer Verbindung mit der Struktur

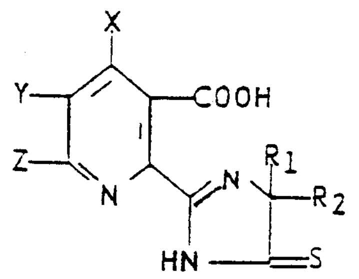

wobei $R_1$ , $R_2$, X, Y und Z wie in Anspruch 1 beschrieben sind, mit mindestens einem molaren Äquivalent Trifluoressigsäureanhydrid.

3. Verbindung mit der Struktur

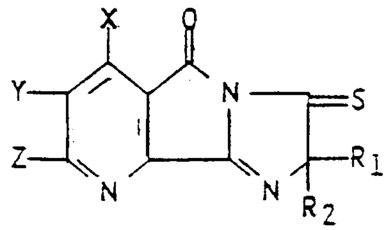

wobei $R_1$ , $R_2$, X, Y und Z wie in Anspruch 1 definiert sind.

4. Verfahren zur Herstellung einer Verbindung gemäß Anspruch 3, umfassend die Umsetzung einer Verbindung mit der Struktur

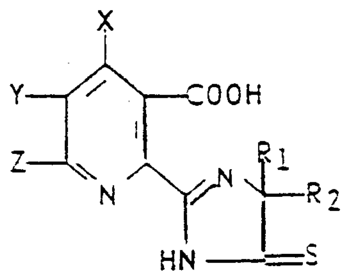

wobei $R_1$ , $R_2$, X, Y und Z wie in Anspruch 3 beschrieben sind, mit mindestens einem molaren Äquivalent von Dicyclohexylcarbodiimid.

5. Verwendung einer Verbindung, wie sie in Anspruch 3 definiert ist, zur Herstellung von herbiziden Verbindungen mit der Struktur

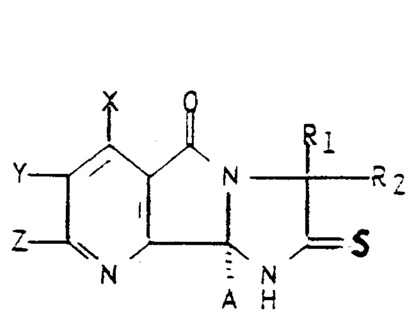

wobei $R_1$ $R_2$, X, Y und Z wie in Anspruch 3 definiert sind;

A für Hydroxyl, $C_{3-6}$-Alkenyloxy, $C_{3-6}$-Alkinyloxy, $C_{3-6}$-Alkylthio, $NR_{13}R_{14}$ oder $C_{1-6}$-Alkoxy, gegebenenfalls substituiert mit Phenyl, Halogenphenyl, $C_{1-3}$-Alkylphenyl, $C_{1-3}$-Alkoxyphenyl oder Di-$C_{1-3}$-alkylaminophenyl steht;

$R_{13}$ für Wasserstoff, $C_{1-4}$-Alkyl, gegebenenfalls substituiert mit Phenyl, Halogenphenyl, $C_{1-3}$-Alkylphenyl oder $C_{1-3}$-alkoxyphenyl, steht; und

$R_{14}$ für Wasserstoff oder $C_{1-4}$-Alkyl steht.

## Revendications

**1.** Composé ayant la structure

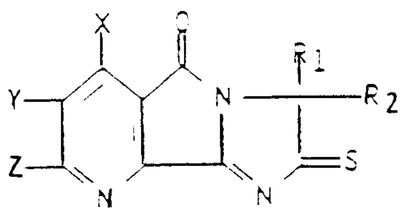

dans laquelle

$R_1$ et $R_2$ représentent chacun un groupe alkyle en $C_1$-$C_3$ ou cyclopropyle, sous réserve que la somme du nombre des atomes de carbone de $R_1$ et de $R_2$ soit de 2 à 5 ; et $R_1$ et $R_2$, ensemble avec l'atome de carbone auquel ils sont fixés, peuvent former un cycle cycloalkyle en $C_3$-$C_6$ facultativement substitué par un groupe méthyle ;

X est un atome d'hydrogène ou d'halogène ou un groupe méthyle ;

Y et Z sont chacun un atome d'hydrogène ou d'halogène ou un groupe alkyle en $C_1$-$C_6$, hydroxyalkyle en $C_1$-$C_4$, alcoxy en $C_1$-$C_6$, alkylthio en $C_1$-$C_4$, phénoxy, halogénoalkyle en $C_1$-$C_4$, $OCF_2CHF_2$, $OCF_3$, $OCHF_2$, nitro, cyano, $NR_4R_5$, alcényloxy droit ou ramifié en $C_3$-$C_8$ facultativement substitué par 1 à 3 atomes d'halogène, alcynyloxy droit ou ramifié en $C_3$-$C_8$ facultativement substitué par 1 à 3 atomes d'halogène, ou phényle facultativement substitué par un groupe alkyle en $C_1$-$C_4$ ou alcoxy en $C_1$-$C_4$ ou un atome d'halogène ;

$R_4$ est un atome d'hydrogène ou un groupe alkyle en $C_1$-$C_4$ ;

$R_5$ est un groupe alkyle en $C_1$-$C_4$ ;

et, ensemble, Y et Z peuvent former un cycle dans lequel YZ est représenté par

(1) la structure : $-(CH_2)_n-$, dans laquelle n est un entier valant 2, 3 ou 4, sous réserve que X soit un atome d'hydrogène ; ou

(2) la structure :

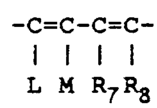

dans laquelle L, M, $R_7$ et $R_8$ représentent chacun un atome d'hydrogène ou d'halogène ou un groupe alkyle en $C_1$-$C_4$, alcoxy en $C_1$-$C_4$, alkylthio en $C_1$-$C_4$, alkylsulfonyle en $C_1$-$C_4$, halogénoalkyle en $C_1$-$C_4$, $NO_2$, CN, phényle, phénoxy, amino, $OCF_3$, $OCHF_2$, $OCF_2CHF_2$, alkylamino en $C_1$-$C_4$, dialkyl($C_1$-$C_4$)amino, chlorophényle, méthylphényle, alcényloxy droit ou ramifié en $C_3$-$C_8$ facultativement substitué par 1 à 3 atomes d'halogène, alcynyloxy droit au ramifié en $C_3$-$C_8$ facultativement substitué par 1 à 3 atomes d'halogène, ou phénoxy substitué par un groupe Cl, $CF_3$, $NO_2$ ou $CH_3$, sous reserve qu'un seul de L, M, $R_7$ ou $R_8$ puisse représenter un substituant autre qu'un atome d'hydrogène ou d halogène ou un groupe alkyle en $C_1$-$C_4$ ou alcoxy en $C_1$-$C_4$ ; ou

(3) les structures :

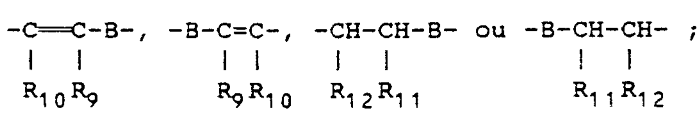

dans lesquelles B est un atome d'oxygène ou de soufre ; $R_9$ et $R_{10}$ représentent chacun un atome d'hydrogène ou d'halogène, ou un groupe phényle ou alkyle en $C_1$-$C_4$ ; $R_{11}$ et $R_{12}$ représentent chacun un atome d'hydrogène ou un groupe alkyle en $C_1$-$C_4$ ou phényle ;

et, lorsque $R_1$ et $R_2$ ne sont pas semblables, leurs isomères optiques.

**2.** Procédé pour préparer un composé selon la revendication 1 comprenant la réaction d'un composé ayant la structure

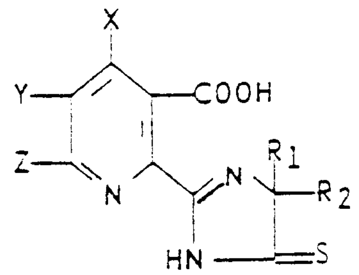

dans laquelle $R_1$, $R_2$, X, Y et Z sont comme décrits dans la revendication 1, avec au moins un équivalent molaire d'anhydride trifluoroacétique.

**3.** Composé ayant la structure

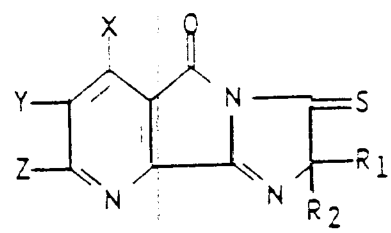

dans laquelle $R_1$, $R_2$, X, Y et Z sont comme définis dans la revendication 1.

**4.** Procédé pour préparer un composé selon la revendication 3 comprenant la réaction d'un composé ayant la structure

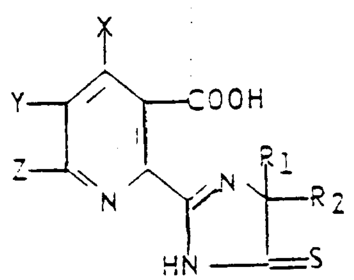

dans laquelle $R_1$, $R_2$, X, Y et Z sont comme décrits dans la revendication 3, avec au moins un équivalent molaire de dicyclohexylcarbodiimide.

**5.** Utilisation d'un composé comme défini dans la revendication 3 pour préparer des composés herbicides ayant la structure

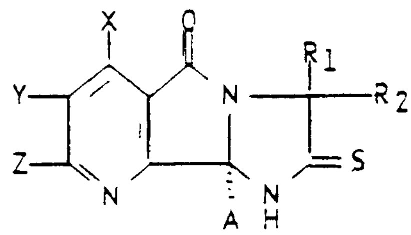

dans laquelle $R_1$, $R_2$, X, Y et Z sont comme définis dans ladite revendication 3 ;
A est un groupe hydroxy, alcényloxy en $C_3$-$C_6$, alcynyloxy en $C_3$-$C_6$, alkylthio en $C_1$-$C_6$, $NR_{13}R_{14}$ ou alcoxy en $C_1$-$C_6$ facultativement substitué par un groupe phényle, halogénophényle, alkyl($C_1$-$C_3$)-phényle, alcoxy($C_1$-$C_3$)phényle ou dialkyl($C_1$-$C_3$)aminophényle ;
$R_{13}$ est un atome d'hydrogène, un groupe alkyle en $C_1$-$C_4$ faculativement substitué par un groupe phényle, halogénophényle, alkyl($C_1$-$C_3$)phényle ou alcoxy($C_1$-$C_3$) phényle ; et
$R_{14}$ est un atome d'hydrogène ou un groupe alkyle en $C_1$-$C_4$.